(19) Europäisches Patentamt
European Patent Office
Office européen des brevets

(11) Veröffentlichungsnummer: **0 174 458**
**A1**

# (12) EUROPÄISCHE PATENTANMELDUNG

(21) Anmeldenummer: **85108578.7**

(22) Anmeldetag: **10.07.85**

(51) Int. Cl.⁴: **A 61 K 31/54**
C 07 D 279/16, C 07 D 279/20
C 07 D 279/22, C 07 D 279/14
C 07 D 513/04, C 07 D 417/04
C 07 D 417/06, C 07 F 7/08
//A61K31/00, C07D279/00,
C07D513/00, C07D417/00,
C07F7/00, (C07D513/04, 311:00,
279:00), (C07D513/04, 307:00,
279:00), (C07D513/04, 279:00,
209:00), (C07D513/04, 335:00,
279:00), (C07D513/04, 279:00,
221:00)

(30) Priorität: **19.07.84 DE 3426564**

(43) Veröffentlichungstag der Anmeldung:
**19.03.86 Patentblatt 86/12**

(84) Benannte Vertragsstaaten:
**AT BE CH DE FR GB IT LI NL SE**

(71) Anmelder: **BAYER AG**
**Konzernverwaltung RP Patentabteilung**
**D-5090 Leverkusen 1 Bayerwerk(DE)**

(72) Erfinder: **Benz, Günter, Dr.**
**Am Bölkumer Busch 5**
**D-5620 Velbert 15(DE)**

(72) Erfinder: **Fengler, Gerd, Dr.**
**Bethelstrasse 16**
**D-4150 Krefeld(DE)**

(72) Erfinder: **Meyer, Horst, Dr.**
**Henselweg 11**
**D-5600 Wuppertal 1(DE)**

(72) Erfinder: **Niemers, Ekkehard, Dr.**
**In den Birken 51a**
**D-5600 Wuppertal 1(DE)**

(72) Erfinder: **Fiedler, Volker, Dr.**
**Lehner Mühle 46**
**D-5090 Leverkusen 3(DE)**

(72) Erfinder: **Mardin, Mithat, Dr.**
**153 Horse Pond Road**
**Madison, CT 06443(US)**

(72) Erfinder: **Mayer, Dieter, Prof. Dr.**
**Beethovenstrasse 1**
**D-4712 Werne(DE)**

(72) Erfinder: **Perzborn, Elisabeth, Dr.**
**Am Tescher Busch 13**
**D-5600 Wuppertal 11(DE)**

(72) Erfinder: **Seuter, Friedel, Dr.**
**Moospfad 16**
**D-5600 Wuppertal 1(DE)**

(72) Erfinder: **Hammond, Michael, Dr.**
**3 London Road**
**Postcombe Oxon(GB)**

(72) Erfinder: **Taylor, William A., Dr.**
**"Wyndin" The Willows Maidenhead Road**
**Windsor Berks(GB)**

(54) **Verwendung von 4H-1,4 Benzothiazinen bei der Prophylaxe und Therapie von Erkrankungen der Atemwege, Entzündungen/Rheuma, thromboembolischen Erkrankungen, Ischämien und Infarkten, Herzrhythmusstörungen, Arteriosklerose und Dermatosen, Arzneimittel zu diesem Zweck sowie Wirkstoffe, die in diesen Arnzeimitteln enthalten sind.**

(57) 4H-1,4-Benzothiazine können zur Prophylaxe und Therapie von Erkrankungen der Atemwege, Entzündungen, Rheuma thromboembolischen Erkrankungen, Ischämien und Infrakten, Herzrthythmusstörungen, Arteriosklerosen und Dermatosen verwendet werden.

Die erfindungsgemäß verwendbaren 4H-1, 4-Benzothiazine sind zum Teil neu und entsprechen der allgemeinen Formel I

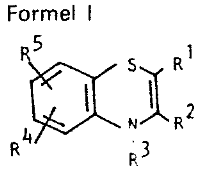

(I)

EP 0 174 458 A1

BAYER AKTIENGESELLSCHAFT 5090 Leverkusen, Bayerwerk
Konzernverwaltung RP
Patentabteilung Ad/Kü-c

# Verwendung von 4H-1,4-Benzothiazinen bei der Prophylaxe und Therapie von Erkrankungen der Atemwege, Entzündungen/Rheuma, thromboembolischen Erkrankungen, Ischämien und Infarkten, Herzrhythmusstörungen, Arteriosklerose und Dermatosen, Arzneimittel zu diesem Zweck sowie Wirkstoffe, die in diesen Arzneimitteln enthalten sind

---

Die vorliegende Erfindung betrifft die Verwendung von 4H-1,4-Benzothiazinen als Hemmer/Stimulatoren von enzymatischen Reaktionen im Rahmen des Arachidonsäurestoffwechsels. Derartige Substanzen sind zur Verhütung und Behandlung von Erkrankungen der Atemwege wie Allergie/Asthma, Bronchitis, Emphysem, Schocklunge, pulmonaler Hypertonie, Entzündungen/Rheuma und Ödemen, Thrombosen und Thromboembolien, Ischämien (periphere, kardiale, cerebrale Durchblutungsstörungen), Herz- und Hirninfarkten, Herzrhythmusstörungen, Angina pectoris, Arteriosklerose, bei Gewebetransplantationen, Dermatosen wie Psoriasis und zur Cytoprotektion im Gastrointestinaltrakt geeignet.

Gegenstand der Erfindung sind auch Arzneimittel für den vorgenannten Verwendungszweck, welche durch den Gehalt an einer therapeutisch wirksamen Menge von 4H-1,4-Benzothiazinen gekennzeichnet sind.

Die erfindungsgemäßen Verbindungen sind bevorzugt Hemmer der Lipoxygenase und stimulieren gleichzeitig die Prostacyclinsynthese. Bekannte Lipoxygenasehemmer wie Nordihydroguaretsäure, 3-Amino-1-(3-trifluormethylphenyl)-pyrazolin (BW 755C), Phenidon und 5,8,11,14-Eikosatetrainsäure sind entweder gleichzeitig als Cyclooxygenasehemmer oder erst bei sehr hohen Konzentrationen wirksam. Die Hemmung des Enzyms Cyclooxygenase des Arachidonsäuremetabolismus führt zu einer globalen Prostaglandinsynthesehemmung und im allgemeinen zu einer Stimulation des Lipoxygenaseweges, was Gastrotoxizität und pro-inflammatorische und asthmatische Wirkungen sowie eine erhöhte Thrombose- und Arterioskleroseneigung verursachen kann.

Die erfindungsgemäß verwendbaren 4H-1,4-Benzothiazine sind zum Teil neu und entsprechen der allgemeinen Formel I

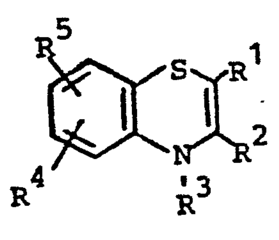

(I)

worin

$R^1$ für Wasserstoff, Aryl (gegebenenfalls substituiertes), Hetaryl (gegebenenfalls substituiertes), Alkyl (gegebenenfalls substituiertes), Cyano, $-CO-R^6$, wobei $R^6$ und $R^2$ durch Brückenglieder verbunden sein können,

$R^2$ für Wasserstoff, Deuterium, Alkyl (gegebenenfalls substituiertes), Aryl (gegebenenfalls substituiertes), $-CH_2-R^6$, $-CO-R^6$, wobei $R^6$ und $R^1$ durch Brückenglieder verbunden sein können,

$R^6$ und $R^2$ bzw. $R^6$ und $R^1$ für Alkylen mit 2 bis 4 C-Atomen steht, das ein oder mehrfach substituiert sein kann, durch Alkyl, Alkoxy, Aryl, Fluor, Alkoxycarbonyl, wobei eine Methylengruppe auch durch O, S, SO, $SO_2$, NH oder N-Alkyl ersetzt sein kann,

$R^3$ für Wasserstoff, Alkyl (gegebenenfalls substituiertes), Acyl

$R^4$, $R^5$ für Wasserstoff, Alkyl (gegebenenfalls substituiertes z.B. Trifluormethyl), Halogen, Alkoxy, Nitro, Acyloxy, Cyano, N-Alkylpiperazino, Alkylsulfonyl, Arylsulfonyl, Amidosulfonyl, Carboxyl und wobei $R^4$ und $R^5$ gleich oder verschieden sein können,

$R^6$ für Wasserstoff, Alkyl (gegebenenfalls substituiertes), Aryl (gegebenenfalls substituiertes), Cycloalkyl, Amino (gegebenenfalls substituiertes), gegebenenfalls substituiertes Alkoxy, Cycloalkoxy, Alkoxycarbonyl, Thioalkyl, Thiophenyl (gegebenenfalls substituiert),

steht.

Die erfindungsgemäß verwendeten 1,4-Benzothiazine entsprechen bevorzugt der allgemeinen Formel Ia

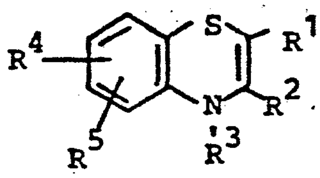

(Ia)

worin

$R^1$ für Wasserstoff, Aryl (gegebenenfalls substituiertes), Hetaryl (gegebenenfalls substituiertes) oder

$-\overset{O}{\overset{\|}{C}} - R^6$, $C \equiv N$ steht, wobei $R^6$ und $R^2$ durch Brückenglieder verbunden sein können,

$R^2$ für H; Alkyl (gegebenenfalls substituiertes)

$R^3$ für H;

$R^4$, $R^5$ für H; Alkyl (gegebenenfalls substituiertes); Halogen; Alkoxy; $NO_2$; Acyloxy, Cyano, Alkylpiperazino, Alkylsulfonyl; Arylsulfonyl, Amidosulfonyl, Carboxyl steht und wobei $R^4$ und $R^5$ gleich oder verschieden sein können,

$R^6$ für Amino (gegebenenfalls substituiertes), Thioalkyl, Thiophenyl, Cycloalkoxy, substituiertes Methoxy der Formel $-O-CH_2-R^8$, steht und

$R^8$ für H oder gegebenenfalls substituiertes Alkyl oder gegebenenfalls substituiertes Aryl steht.

In der allgemeinen Formel I steht gegebenenfalls substituiertes Alkyl, $R^1$, $R^2$, $R^3$, $R^4$, $R^5$ und $R^6$ für geradkettiges oder verzweigtes Alkyl mit vorzugsweise 1 bis 8, besonders bevorzugt 1 bis 4 Kohlenstoffatomen. Beispielhaft seien besonders bevorzugt gegebenenfalls substituiertes Methyl, Ethyl, n- und i-Propyl, n-, i- und t-Butyl genannt.

Die Alkylreste $R^1$ bis $R^6$ können 1- bis 3-mal, vorzugsweise einmal, durch folgende Substituenten substituiert

sein: Halogen, insbesondere Fluor und/oder Chlor, Dialkylamino, Pyrrolidino, Piperidino, gegebenenfalls substituiertes Aryl, Alkoxy, Alkylthio, Alkylsulfinyl, Alkylsulfonyl, $-CO-R^7$, wobei $R^7$ für Alkyl, gegebenenfalls substituiertes Aryl, Hydroxy, Alkoxy, Amino, Alkylamino, Dialkylamino, Pyrrolidino, Piperidino und Morpholino steht.

Als gegebenenfalls substituiertes Aryl $R^1$, $R^2$, $R^6$, $R^7$, $R^8$ und Substituent der Alkylreste $R^1$ bis $R^6$ und $R^8$ steht Aryl mit vorzugsweise 6 bis 20 Kohlenstoffatomen im Arylteil. Beispielhaft seien Phenyl oder Naphthyl genannt. Die Arylreste können 1- bis 3-mal, vorzugsweise einmal in o-, m- oder p-Stellung durch folgende Substituenten substituiert sein: Halogen, Alkyl, Alkoxy, Trifluormethyl, Nitro.

Als gegebenenfalls substituiertes Hetaryl $R^1$ steht besonders bevorzugt 2-Thienyl, 3-Thienyl, 2-Furyl, 3-Furyl, 2-Pyridyl, 3-Pyridyl, 4-Pyridyl, 2-Pyrimidyl, 4-Pyrimidyl, 5-Pyrimidyl. Diese Reste können durch Alkyl, Alkoxy, Hydroxy oder Halogen, insbesondere Fluor und/oder Chlor, ein- oder mehrfach substituiert sein.

Als gegebenenfalls substituiertes Alkoxy-$R^6$ steht geradkettiges oder verzweigtes Alkoxy mit vorzugsweise 1 bis 8, besonders bevorzugt 1 bis 4 Kohlenstoffatomen.

Die Alkoxyreste können 1- bis 3-mal, vorzugsweise einmal, durch folgende Substituenten substituiert sein: Halogen, insbesondere Fluor und/oder Chlor, Aryl (gegebenenfalls substituiert), Hydroxy, Alkoxy, Alkylthio, Cyano.

Als gegebenenfalls substituiertes Alkyl $R^8$ steht geradkettiges oder verzweigtes Alkyl mit vorzugsweise 1 bis 7 Kohlenstoffatomen. Die Alkylreste $R^8$ können 1- bis 3-mal, vorzugsweise einmal, durch folgende Substituenten substituiert sein: Halogen, insbesondere Fluor und/oder Chlor, Aryl (gegebenenfalls substituiertes), Hydroxy, Alkoxy, Alkylthio, Cyano.

Als gegebenenfalls substituiertes Amino-$R^6$ steht Monoalkylamino, Dialkylamino, Pyrrolidino, Piperidino, Morpholino.

Die erfindungsgemäß verwendeten Verbindungen der allgemeinen Formel I lassen sich gemäß der Verfahrensvariante 1 herstellen, indem man 2-Aminothiophenole der Formel II mit Verbindungen der Formel III in Dimethylsulfoxid zur Reaktion bringt [vgl. J. Chem. Soc. Perkin Trans. I, 1146-49 (1976); Synthesis 933-55 (1983)].

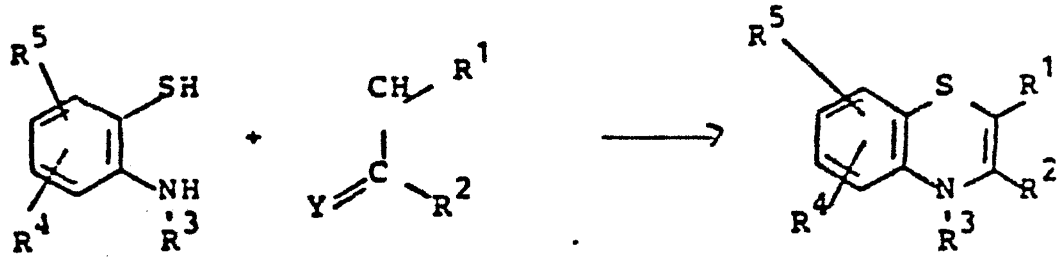

II III I

Die Verbindungen der Formeln II und III sind entweder literaturbekannt oder lassen sich in Analogie zu literaturbekannten Verbindungen herstellen. Die Reaktionstemperatur kann zwischen 50°C und 200°C liegen, vorzugsweise zwischen 100°C und 150°C. In einigen Fällen ist es zweckmäßig unter Sauerstoffausschluß zu arbeiten.

Die erfindungsgemäß verwendeten Stoffe lassen sich nach Verfahrensvariante 2 herstellen, indem man 2-Aminothiophenole der Formel II mit Verbindungen der Formel IV zur Reaktion bringt (vgl. Ann. Chem. 744, 20-32 (1971)).

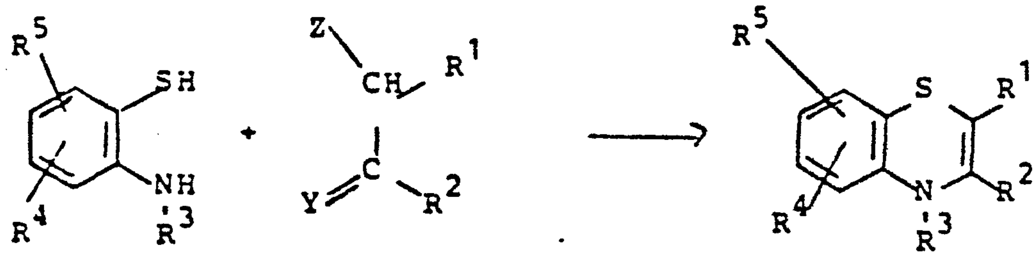

II IV I

y = Sauerstoff; Alkyl-N; Aryl-N
z = Halogen, vorzugsweise Cl, Br.

Die Reaktion kann vorzugsweise auch in Gegenwart eines Säurefängers, wie z.B. Triethylamin, $K_2CO_3$, $Na_2CO_3$, NaOH, $NH_3$ durchgeführt werden.

Als Lösungsmittel kommen in Frage: Alkohole (z.B. Methanol, Ethanol), Ether (z.B. Diethylether, THF, Dioxan, Dimethoxyethan, Diethylenglykoldimethylether), Kohlenwasserstoffe (z.B. Benzol, Toluol), chlorierte Kohlenwasserstoffe (z.B. Methylenchlorid, Chloroform, Chlorbenzol), DMSO, DMF, Pyridin und Wasser. In einigen Fällen ist es zweckmäßig unter Sauerstoffausschluß zu arbeiten.

Die Reaktionstemperatur kann zwischen 0°C und 200°C liegen, vorzugsweise zwischen 35°C und 110°C.

Die erfindungsgemäß verwendeten Stoffe lassen sich nach Verfahrensvariante 3 herstellen, indem man Disulfide der Formel V mit Carbonylverbindungen der Formel III zur Reaktion bringt (vergl. Phosphorus and Sulfur 3, 309-314, (1977); Tetrahedron Letters 3795-6 (1980)].

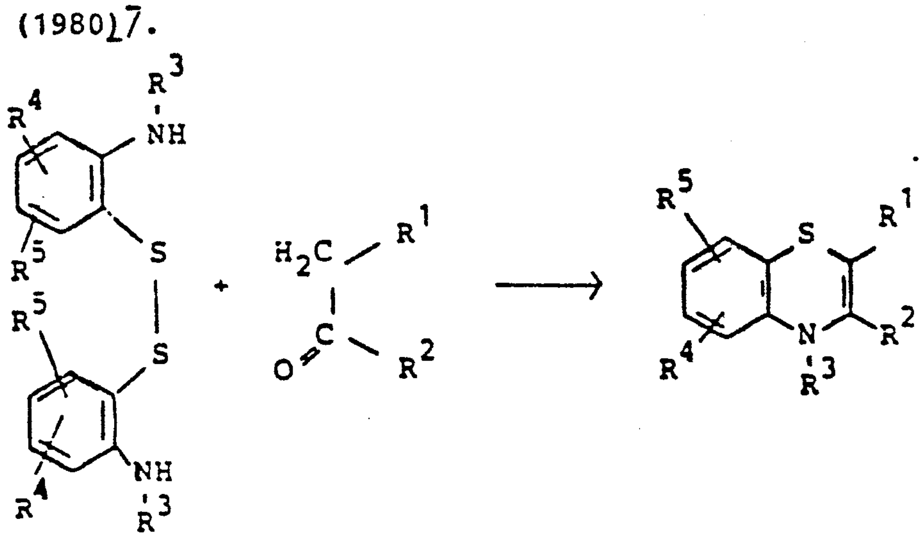

V III I

Die Reaktion wird durch Säuren (z.B. p-Toluolsulfonsäure) katalysiert.

Als Lösungsmittel kommen in Frage: Benzol, Toluol, Pyridin, DMSO und DMF.

Die Reaktionstemperatur kann zwischen 20°C und 180°C liegen, vorzugsweise zwischen 35°C und 110°C.

Es ist zweckmäßig unter Sauerstoffausschluß zu arbeiten.

Die Reaktion wird meist mit äquimolaren Mengen der Ausgangsmaterialien durchgeführt, gelegentlich wird auch ein Überschuß (bis zu 110 %) an Carbonylverbindung verwendet.

Die erfindungsgemäß verwendeten Stoffe der Formel I lassen sich nach Verfahrensvariante 4 herstellen ($R^6$ und $R^2$ sind nicht überbrückt), indem man Disulfide der Formel V mit Alkinen der Formel VI zur Reaktion bringt [vgl. J. Heterocyclic Chem. 17, 377 (1980)].

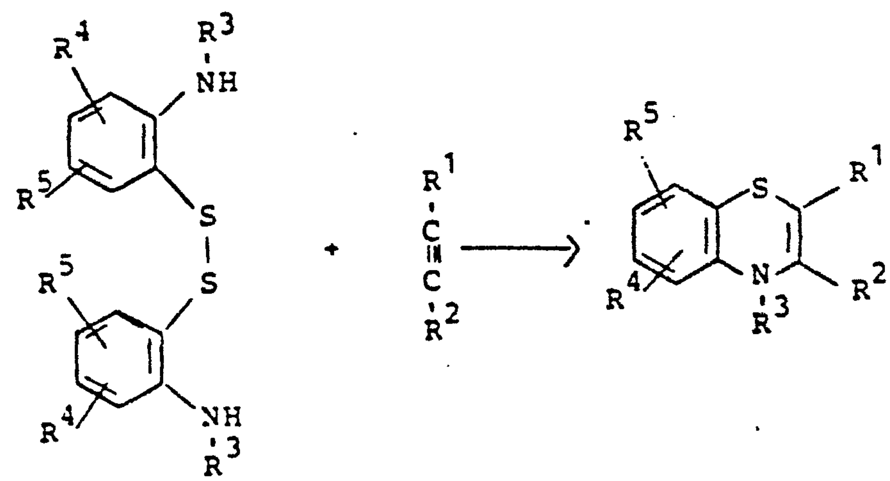

V VI I

Die Reaktion wird vorzugsweise mit äquimolaren Mengen der Ausgangsmaterialien durchgeführt, gelegentlich wird auch ein Überschuß (bis zu 100 %) an Alkin (VI) verwendet. Als Lösungsmittel kommen in Frage: Alkohole (z.B. Methanol, Ethanol), Ether, Kohlenwasserstoffe (z.B. Benzol, Toluol), chlorierte Kohlenwasserstoffe, DMSO, DMF und Pyridin. Die Reaktionstemperatur liegt zwischen 20°C und 180°C, vorzugsweise zwischen 35°C und 120°C.

Es ist zweckmäßig unter Sauerstoffausschluß zu arbeiten.

Erfindungsgemäß verwendete Stoffe der Formel I, bei denen $R^3$ für Alkyl (gegebenenfalls substituiertes) steht, lassen sich aus den entsprechenden Verbindungen, bei denen $R^3$ für Wasserstoff steht, nach den üblichen Alkylierungsmethoden herstellen, z.B. durch Umsetzung mit $CH_3J$ oder $C_2H_5J$.

Bekannte Lipoxygenasehemmer wie 3-Amino-1-(m-trifluormethylphenyl)-pyrazolin-2 zeigen bei systemischer Verabreichung (z.B. oral) toxische Nebenwirkungen. Es besteht deshalb ein Bedarf nach stärker wirksamen Verbindungen mit einem selektiveren Wirkungsprofil ohne Nebenwirkungen.

Überraschenderweise entsprechen die 4H-1,4-Benzothiazine dem geforderten pharmakologischen Profil, was in mehreren für die beanspruchten Wirkungen relevanten Modellen nachgewiesen wurde.

Die erfindungsgemäß einzusetzenden 4H-1,4-Benzothiazine weisen auch lokal gefäßdilatierende Effekte auf, die besonders im ischämischen Gewebe (z.B., Hirn, Peripherie) von therapeutischem Nutzen sind. Im Unterschied zu Indometacin, welches die Prostaglandinsynthese aus der Arachidonsäure insgesamt hemmt, greifen die erfindungsgemäß einzusetzenden 4H-1,4-Benzothiazine viel spezifischer in den Stoffwechsel der für die Bildung des $PGI_2$, des Thromboxans und der Leukotriene maßgebenden Enzyme ein, so daß nicht nur der schädigende, gefäßverengende und arrythmienerhöhende Einfluß des Thromboxans und der Leukotriene reduziert, sondern auch der gefäßerweiternde Einfluß des Prostacylins durch Stimulierung seiner Bildung erhöht wird.

Die biologische Wirkung der patentgemäß hergestellten Verbindungen wurde durch folgende Experimente nachgewiesen:

A) <u>Lipoxygenasehemmung/Cyclooxygenasehemmung/Prostacyclinstimulation</u>

1. <u>Menschliche PMN-Leukozyten</u>

Selektiv wird die für die Biosynthese der Leukotriene $B_4$, $C_4$ und $D_4$ verantwortliche 5-Lipoxygenase bei niedrigen Konzentrationen gehemmt.

Die polymorphkernigen Leukozyten des Menschen metabolisieren die Arachidonsäure zu 5-Hydroxy-6,8,11,14-eikosatetraensäure (5-HETE) und Leukotrien $B_4$ (5S, 12R-Dihydroxy-6 cis, 8,10-trans-14 cis-eikosatetraensäure). Die Hemmung der Freisetzung von 5-HETE und Leukotrien $B_4$ aus den Leukozyten stellt ein Maß für den lipoxygenasehemmenden Effekt der erfindungsgemäßen Verbindungen dar. (Vergl. Tabelle 1).

Der Test mit den Humanleukozyten wurde nach Borgeat und Samuelsson (J. Biol. Chem. 254, 2643, 1979 und Proc. Natl. Acad. Sci. USA, 76, 2148, 1979) durchgeführt.

Die im vorliegenden Beispiel verwendeten Human PMN-Leukozyten (> 95 %) wurden aus heparinisiertem Vollblut durch eine Dextran-Sedimentation und anschließende Dichtegradiententrennung (Ficoll-Paque) gewonnen (vgl. A. Boyum, Scand. J. Immunol., 5, Suppl 5, 9, 1976).

2 x $10^7$ Zellen/ml wurden in $Ca^{2+}$-haltigem Dulbecco-Phosphat-Puffer suspendiert und in Anwesenheit bzw. Abwesenheit von Lipoxygenasehemmer mit dem Calcium-Ionophor A 23187 stimuliert.

Nach 15 Minuten wurden die Lipoxygenaseprodukte aus dem angesäuerten Inkubationsmedium extrahiert und mittels HPLC getrennt. Die Hochdruckflüssigkeitschromatographie wurde auf Lichrosorb RP-18(5 µm)-Säulen durchgeführt. Das Laufmittel war Methanol/Wasser/Eisessig 69/31/0.01. Die Flußrate betrug 1 ml/min. Leukotrien $B_4$ wurde bei 280 nm, die HETE-Derivate bei 232 nm mittels der UV-Absorption bestimmt.

2. $^{3}H$-Arachidonsäuremetabolismus in menschlichen Thrombozyten (12-Lipoxygenaseaktivität und Cyclooxygenaseaktivität)

Der Nachweis der lipoxygenasehemmenden und cyclooxygenasehemmenden Eigenschaften der erfindungsgemäßen Verbindungen erfolgte analog der Methode von Bailey et al., Journal of Biol. Cemistry 255, 5996, 1980 und nach Blackwell und Flower, Prostaglandins 16, 417, 1978. Bei dieser Testmethode wird der Metabolismus radioaktiv markierter Arachidonsäure an gewaschenen Humanthrombozyten herangezogen. Bei diesem in vitro Test werden die radioaktiv markierten Metaboliten aus dem Reaktionsansatz extrahiert und dünnschichtchromatographisch getrennt. Das Autoradiogramm wird am Dünnschichtscanner ausgewertet. Bei diesen Testbedingungen werden die markierten Metaboliten von der nicht umgesetzten Arachidonsäure getrennt

und sind anschließend quantitativ auswertbar. Die Verteilung der Radioaktivität auf die während der Metabolisierung gebildeten Cyclooxygenaseprodukte Thromboxan $A_2$ (bestimmt als $TXB_2$ ) und 12-Hydroxy-5,8,10-heptadekatriensäure (HHT) bzw. das Lipoxygenaseprodukt 12-Hydroxy-5,8,10,11,14-eikosatetraensäure (12-HETE) unter dem Einfluß der Inhibitoren stellt ein Maß für die Hemmung der Enzyme dar. Die Ergebnisse sind in Tabelle 1 dargestellt.

3. Prostacyclinstimulation

Außerdem stimulieren die erfindungsgemäß zu verwendenden 4H-1,4-Benzothiazine spezifisch die Synthese von Prostacyclin ($PGI_2$). $PGI_2$ wirkt im Gegensatz zu dem vasokonstriktorischen und thrombozytenaggregationsauslösenden Thromboxan gefäßdilatierend und thrombozytenaggregationshemmend.

a) Stimulation im Mikrosomen in vitro

Die spezifische $PGI_2$-stimulierende Wirkung wurde in vitro in einem Gemisch aus Mikrosomen von Schafssamenblasen (RSVM) und Rinderaorten (BAM) gezeigt (vgl. Fl. Cottee et al., Prostaglandins, 14, 413, 1977). $^3$H-Arachidonsäure wurde

in Gegenwart der Benzothiazine mit einem Gemisch aus RSVM und BAM 10 Minuten bei 25°C inkubiert. Die Reaktion wurde durch Ansäuern auf pH 3,5 gestoppt. Die Fettsäuremetaboliten wurden mit Essigester extrahiert. Der Essigester wurde unter $N_2$ abgedampft und der Rückstand in $CH_3OH/CHCl_3$ (1:1) aufgenommen und auf DC-Plastikfolien aufgetragen. Die Trennung erfolgte mit einem Fließmittelgemisch Ethylacetat/Eisessig/Isooctan/$H_2O$ (110:20:50:10; organische Phase) (P. Needleman et al., The Journal of Clinical Investigation 1978, 61, 839-849). Die Verteilung der Radioaktivität wurde mittels eines Radioscanners gemessen.

Die Tabelle 1 zeigt den Einfluß mehrerer Benzothiazine auf die Synthese von Prostacyclin in RSVM gegenüber dem Kontrollversuch ohne Wirkstoff.

b) Stimulation in Aortenringen ex vivo.

Aortenringe von mit Prüfsubstanzen vorbehandelten Ratten werden 10 Minuten bei 37°C in Trispuffer (pH 7,8) inkubiert. Das sich bildende Prostacyclin wird nach

Umwandlung zu dem stabilen 6-Keto-PGF$_{1\alpha}$ radioimmunologisch bestimmt.
Literatur:
Mc Intyre, D.E., Salzman, E.W., Thrombos. Haemostas. 46, 19, 1981.
Seuter, F. et al., 6th International Symposium on Atherosclerosis, Abstr. 270, Berlin 1982.

B) Leukozytenklebrigkeit

Überraschenderweise wurde auch gefunden, daß die erfindungsgemäß einzusetzenden 4H-1,4-Benzthiazine die Klebrigkeit von Leukozyten (stickiness) vermindern, d.h. deren Wanderungsgeschwindigkeit erhöhen. Die Verminderung der Leukozytenklebrigkeit trägt ebenfalls zur Abnahme ischämischer Gebiete bei, denn die Leukozyten passieren nach Behandlung mit den erfindungsgemäß einzusetzenden 4H-1,4-Benzthiazinen den mikrozirkulatorischen Bereich dieser Gebiete, ohne durch totalen oder zeitweiligen Verschluß von kleinen Gefäßen bzw. Kapillaren eine Minderversorgung des entsprechenden Gewebes hervorzurufen.

Die Zahl der Leukozyten, die ein Gefäß in einem bestimmten Abschnitt passieren, dient als einfaches Modell zur Messung der Klebrigkeit von Leukozyten (Bray, M.A. et al.., Prostaglandins 22, 312, 1981). Die Anzahl der gezählten Leukozyten wird umso größer, je geringer die Klebrigkeit dieser Zellen wird.

Männliche Syrische Goldhamster (80-100 g) wurden mit Nembutal (i.p.; 60 mg/kg) narkotisiert. Nach Einbinden eines PE 10 Katheters in die A. femoralis wird das Tier auf eine Präparierbühne nach Duling (MVR 5, 423, 1973) gelegt und die rechte Backentasche durch Einführen eines Q-Tips herausgezogen. Sie wird vorsichtig in den dafür vorgesehenen Teil der Bühne gezogen, ausgespannt und fixiert.

Ab Beginn der Präparation wird die vorbereitete Backentaschen mit 5 ml/Minute Superfusat überspült. Die Temperatur der Lösung beträgt 36°C. Unter Schonung der Gefäße wird die obere Gewebsschicht längs durchtrennt und zur Seite geklappt. Bei ca. 200facher Vergrößerung wird eine Fläche von ca. 1 $cm^2$ von Bindegewebe vorsichtig freipräpariert. Das Tier wird mit der Präparierbühne auf den Objekttisch des Mikroskops gelegt. Ein Thermofühler wird in die linke Backentasche eingeführt. Die Körpertemperatur des Tieres wird so kontrolliert und mit Hilfe einer IR-Lampe (250 Watt) auf ± 0,5°C gehalten.

Die Messung der Leukozytenklebrigkeit wird bei ca. 500facher Vergrößerung vorgenommen. In dem freipräparierten Feld wird eine Venule (30-40 µm Ø) ausgewählt. In einem bestimmten Abschnitt dieses Gefäßes wird die Anzahl der pro Zeiteinheit (Min.) vorbeiwanderndern Leukozyten gezählt.

C) Thrombozytenaggregationshemmung

Thrombozyten und deren Adhäsion - sowie Aggregationsfähigkeit - sind, besonders im arteriellen Schenkel des Gefäßsystems, ein wesentlicher pathogenetischer Faktor bei der Entstehung von Thrombosen.

Überraschenderweise waren die erfindungsgemäßen Verbindungen auch in diesem Test wirksam, was die Substanzen in Verbindung mit den anderen Eigenschaften als besonders günstig erscheinen läßt.

Für die in vitro Versuche wurde Blut von gesunden Probanden beiderlei Geschlechts verwendet. Als Antikoagulans wurden einem Teil 3,8 %-iger wäßriger Natriumzitratlösung 9 Teile Blut zugemischt. Mittels Zentrifugation erhält man aus diesem Blut plättchenreiches Zitratplasma (PRP) (Literatur: Jürgens/Beller, Klinische Methoden der Blutgerinnungsanalyse; Thieme Verlag, Stuttgart 1959).

Für diese Untersuchungen wurden 0,8 ml PRP und 0,1 ml der Wirkstofflösung bei 37°C im Wasserbad vorinkubiert. Anschließend wurde die Thrombozytenaggregation nach der turbidometrischen Methode (Literatur: Born, B.V.R., J. Physiol. (London), 162, 67, 1962) im Aggregometer bei 37°C bestimmt (Literatur: Therapeutische Berichte 47, 80-86, 1975). Hierzu wurde die vorinkubierte Probe mit 0,1 ml Kollagen, einem aggregationsauslösenden Agens, versetzt.

Die Veränderung der optischen Dichte in der Probe des PRP wurde während einer Zeitdauer von 6 Minuten aufgezeichnet und der Ausschlag nach 6 Minuten bestimmt. Hierzu wird die prozentuale Hemmung gegenüber der Kontrolle errechnet.

Weiterhin wird in Tabelle 4 die prozentuale Hemmung der Thrombozytenaggregation im PRP von Ratten bestimmt. Bei der Durchführung dieser Versuche wurde den Tieren die Wirksubstanz in einer Tylosesuspension oral verabreicht. Anschließend wurden die Tiere 90 Minuten nach der oralen Verabreichung der Substanz entblutet und das PRP mittels Zentrifugation gewonnen. Nach Gewinnung des PRP beginnt in vitro die Messung der Aggregationshemmung analog dem Verfahren, welches für die in vitro Versuche beschrieben ist; jedoch ohne Vorinkubation der Proben. 3 Teile Ratten-PRP wurden außerdem mit einem Teil physiologischer Kochsalzlösung verdünnt.

D) Protektion gegenüber einer durch Arachidonsäure induzierten Thromboembolie

In diesem Modell wirken Substanzen, die mit der Prostaglandin- und Thromboxanbiosynthese interferieren. Kaninchen werden 1,5 mg/kg Na-arachidonat in eine marginale Ohrvene injiziert. Die Tiere sterben innerhalb von Minuten, vermutlich aufgrund vasospastischer Cyclooxygenase- und Lipoxygenaseprodukte sowie embolisierender Plättchenthromben.

Vorbehandlung mit den erfindungsgemäßen Verbindungen schützt die Tiere, so daß diese überleben.

Literatur:

Silver, M.J. et al., Science 183, 1085, 1974
Seuter, F., Busse, W.D., Agents and Actions, Suppl. 4, 175, 1979.

E) Wirkung der Benzothiazinderivate am nach Langendorff perfundierten Kaninchenherzen

Hinzu kommt, daß die erfindungsgemäß einzusetzenden 4H-1,4-Benzothiazine die schädigende Wirkung eines plötzlichen Sauerstoffeinstroms nach einer hypoxischen oder anoxischen Periode reduzieren bzw. ausschalten. Schädigungen durch Reoxygenierung können nach Bypass-Operationen auftreten. Die erfindungsgemäß einzusetzenden 4H-1,4-Benzothiazine werden daher bei derartigen Operationen geeignet sein, Sauerstoffschäden zu verhindern.

Von den Kaninchen (bis ca. 2 kg) wurde das Herz unter Nembutal-Narkose entnommen und an der Aorta sowie an der A. pulmonaris kanüliert. Zur Messung der isovolumetrischen Kontraktion wurde ein flüssigkeitsgefüllter Ballon aus Silikonkautschuk über den linken Vorhof in den linken Ventrikel eingeführt. Über eine Flüssigkeitsbrücke war der Ballon mit einem Flüssigkeitsaufnehmer (Statham P 23 Db) verbunden. Der Perfusionsdruck wurde mit

einem Druckaufnehmer, der vor der Aortenkanüle des Herzens über ein T-Stück mit dem Perfusionssystem verbunden war, aufgenommen.

Die Registrierung von Kontraktionskraft und Perfusionsdruck erfolgte mit dem Schnellschreiber Gould 2600 S. Die Herzen wurden elektrisch stimuliert mit der Frequenz 180/min., Reizdauer 5 msec.

Die Perfusion des Herzens erfolgte mit Krebs-Henseleit-Lösung (KH-Lösung), die mit Karbogen (95 % $O_2$, 5 % $CO_2$) oder mit der Gasmischung 2 % $O_2$, 5,6 % $CO_2$, Rest $N_2$ begast wurde. Das Perfusionsvolumen betrug 20 ml/Min., das über eine Rollenpumpe (Fa. Desaga, 132100) eingestellt war. Die Zugabe der Prüfsubstanz und der Thrombozytensuspensionen erfolgte mit Hilfe von Infusionspumpen (Rollenpumpen, Fa. Braun-Melsungen), wobei die Lösung oder Suspension mit der Fließgeschwindigkeit von 0,2 bzw. 0,1 ml/Min. aus einer Infusionsspritze über einen Taigonschlauch und eine feine Kanüle kurz vor dem Herzen in den zuführenden Schlauch infundiert wurde. Die Endkonzentration der Thrombozyten betrug 1 x $10^6$/ml KH-Lösung. Die aus dem Herzen (Kanüle in A. pulmonalis) fließende Lösung wurde periodisch unter Eiskühlung aufgefangen. Zur Messung der Konzentrationen von Thromboxan als $TXB_2$ und von Prostacyclin als 6-Keto-$PGF_{1\alpha}$, deren Bestimmung mit Hilfe von Radioimmunoassays erfolgte, wurden aliquote Volumenanteile gefriergetrocknet und in einem Zehntel des ursprünglichen Volumens mit Wasser aufgenommen.

F) Koronarthrombose

Narkotisierten Hunden wurde nach linker Brusteröffnung ein Silberdraht in die linke Koronararaterie eingepflanzt. Eine Elektrode wurde unter die Haut eingelegt und schloß den Stromkreis. Für 6 Stunden wurde ein Gleichstrom der geschädigten Arterie zugeführt (1). Dies führte in unbehandelten Kontrollen nach 3.2 Stunden zu einem Gefäßverschluß durch einen Plättchen-Fibrinthrombus. Man schließt das während des Experiments aus dem Abfall des Koronarflusses und typischen Elektrokardiogrammveränderungen.

Benzothiazine wurden ca. 1 Stunde vor Beginn der Stimulierung mit Gleichstrom intraduodenal verabreicht.

Nach Versuchsende wurde das herausgeschnittene Herz mit zwei Lösungen perfundiert: Evans Blau über die Aorta zur Markierung der gesunden, unbeeinflußten Herzgebiete; und Triphenyltetrazolium Chlorid zur Bestimmung des Perfusionsgebietes der verschlossenen Arterie (2). Der Infarkt wurde in diesem Gebiet als ungefärbtes, blasses Gebiet eingeschlossen und konnte entweder durch Planimetrie der Herzscheiben oder durch Herausschneiden aus dem Herzmuskel bestimmt werden.

(1) Romson JL, Haack DW, Lucchesi BR. Electrical induction of coronary artery thrombosis in the ambulatory canine: a model for <u>in vivo</u> evaluation of antithrombotic agents. Thromb. Res. 17, 841-853, 1980.

(2) Romson JL, Bush LR, Haack DW, Lucchesi BR. The beneficial effects of oral ibuprofen on coronary artery thrombosis and myocardial ischemia in the conscious dog. J Pharmacol Exp Ther 215, 271-278, 1980.

G) Herzinfarkt und Herzrhythmusstörungen

Die erfindungsgemäß einzusetzenden 4H-1,4-Benzothiazine vermindern darüber hinaus überraschenderweise die Größe von Herzinfarkten nach Myokardischämien und besitzen positiven Einfluß auf Herzrhythmusstörungen. Auch diese erfindungsgemäßen Verwendungszwecke der erfindungsgemäß einzusetzenden 4H-1,4-Benzothiazine werden durch die aus dem Stand der Technik bekannten Indikationen nicht nahegelegt:

Substanzen zur Therapie von Herzrhythmusstörungen werden nach ihren elektrophysiologischen Wirkungen eingeteilt.

Seit der Klassifizierung von Vaughan Williams (Pharmac. Ther. B 1, 115, 1975) unterscheidet man folgende Klassen:

I) Membranstabilisierende Antiarrhytimika vom
   a) Chinidintyp, z.B. Procain, Ajmalin
   b) Lidocaintyp, z.B. Lidocain, Diphenylhydantoin

II) Beta-Rezeptorenblocker wie z.B. Propranolol u.v.a.

III) Calciumantagonisten wie z.B. Verapamil, Nifedipin u.v.a.

IV) Substanzen mit Verlängerung der Aktionspotentialdauer wie z.B. Amiodaron.

Die erfindungsgemäß einzusetzenden 4H-1,4-Benzothiazine sind weder aufgrund ihrer chemischen Struktur noch ihrer bisher bekannten Wirkungen unter die obigen Gruppen zu subsummieren. Es war aus diesem Grund nicht vorhersehbar, daß die erfindungsgemäß einzusetzenden 4H-1,4-Benzothiazine antiarrhythmische Effekte entfalten. Ebensowenig war aufgrund der antithrombotischen Wirkung zu erwarten, daß die erfindungsgemäß einzusetzenden 4H-1,4-Benzothiazine nach erfolgtem Gefäßverschluß - und zwar unabhängig von der Natur des Koronargefäßverschlusses - die Ausbreitung der Myokardischämie stark einschränken und damit die Infarktgröße vermindern und darüber hinaus zu einer rascheren Heilung und geringeren Kreislaufnebenwirkungen führen. Wie durch Tierversuche gezeigt werden kann, verringern die erfindungsgemäß einzusetzenden 4H-1,4-Benzothiazine die ischämiebedingte Anhebung der ST-Strecke als Infarktzeichen; darüber hinaus steigt die R-Welle des peripheren Extremitäten-EKG's weniger stark an und bildet sich schneller zurück; ST-Intervalle werden weniger als in den Kontrollen verändert, was auf eine bessere Repolarisation der Herzmuskelzellen bei vermindertem Herzinfarkt schließen läßt.

Die Wirkungen verschiedener 4H-1,4-Benzothiazine auf den experimentellen Herzinfarkt wurden im Hund nach akuter Unterbindung einer Koronararterie untersucht. Bei Pentobarbital-narkotisierten Hunden wurde die vordere absteigende linke Koronararterie (LAD) proximal für 6 Stunden unterbunden. Die Arterie wurde nicht reperfundiert. Eine Stunde vor Ligatur wurde das 4H-1,4-Benzothiazin in 1 %iger wäßriger Tylosesuspension intraduodenal als Bolus gegeben. In den Kontrollen wurde gleichermaßen vorgegangen, jedoch ohne Wirkstoffgabe. Die Infarktgröße und das Perfusionsgebiet der LAD wurden durch eine duale Perfusionsmethode mit Evans-Blau und Triphenyltetrazoliumchlorid dargestellt.

H) Erkrankungen des Respirationstraktes (Allergie, Asthma, Bronchitis etc.)

Seit der Entdeckung der "slow reacting substance of anaphylaxis (SRS-A)" wurde ihr eine wichtige Rolle bei asthmatischen Bronchokonstriktionen und bei der Entzündung zugeschrieben. Sie wird über den Lipoxygenaseweg des Arachidonsäurestoffwechsels gebildet und als Leukotrien(e) identifiziert ($LTD_4$, $LTC_4$, $LTE_4$). Entsprechende Inhibitoren sind daher potentielle Antiasthmatika. Die meisten bisher bekannt gewordenen Verbindungen (vgl. vor) erfüllen jedoch nicht die Forderungen bzw. sind therapeutisch nicht verwendbar.

Überraschenderweise zeigen Benzothiazinderivate die gewünschte Eigenschaft, was in den nachfolgenden beschriebenen spezifischen Testen gezeigt wird.

1. Lipoxygenase- und Cyclooxygenaseaktivität in Homogenaten menschlicher Lungen

In diesem Test wird die Umwandlung von $^{14}C$-Arachidonsäure durch die Enzyme Cycloxygenase, 5-Lipoxygenase und 15-Lipoxygenase bestimmt.

Damit wird ergänzend zu den Untersuchungen der enzymatischen Aktivitäten in Leukozyten, Thrombozyten und anderen Geweben die Aktivität in der Lunge, dem Hauptzielorgan allergischer Reaktionen, erfaßt. Die angefallenen Produkte wurden dünnschichtchromatograpisch getrennt und im Scanner quantifiziert. Die erfindungsgemäßen Verbindungen hemmten bei im Vergleich zu Referenzsubstanzen niedrigen Konzentrationen die Lipoxygenase (Tabelle 5).

2. Freisetzung von Mediatoren aus menschlichen Lungen

In Anlehnung an die pathophysiologische Situation (Typ I - Überempfindlichkeitsreaktion) werden Lungen mit Serum passiv sensibilisiert. Zugabe eines entsprechenden, spezifischen Allergens führt zur Freisetzung von Mediatoren der Anaphylaxie wie Histamin, SRS-A, Thromboxan und Prostaglandinen.

Da beim Menschen die Lunge das wichtigste Zielorgan für allergische Reaktionen ist, stellt dieser Test ein relevantes Modell zur Prüfung antiasthmatischer Substanzen dar. Die erfindungsgemäßen Verbindungen führten zu einer selektiven Hemmung der Leukotrienfreisetzung (Tabelle 5); Histamin und Thromboxan wurden nicht oder nur zum Teil beeinflußt.

3. Antigeninduzierte Bronchokonstriktion in vivo (Meerschweinchen)

In diesem System (Konzett-Rössler) werden globale Veränderungen im Widerstand der Lungen gemessen, die während einer Bronchokonstriktion auftreten als Folge unterschiedlichster Stimulantien (Antigen, Histamin, Acetylcholin, SRS-A, $PGF_{2\alpha}$ etc.). In der verwendeten Modifikation werden Meerschweinchen mit Ovalbumin passiv sensibilisiert, Histamin wird durch Mepyramin gehemmt und durch Zugabe des spezifischen Antigens kommt es hauptsächlich zur Freisetzung von SRS-A und Thromboxan. Die Antigen-induzierte Bronchokonstriktion wurde bei niedrigen Dosierungen und verschiedenen Verabreichungsformen (oral, parenteral, Inhalation) signifikant reduziert (Tabelle 6).

4. Passive peritoneale Anaphylaxie (Ratte)

Mit dieser in vivo-Methode wird die Wirkung von Substanzen auf die Freisetzung von Mediatoren, insbesondere die Freisetzung von Leukotrienen, erfaßt. Die passive Sensibilisierung von peritonealen Mastzellen der Ratte mit Anti-Ovalbumin Meerschweinchenserum, führt nach intraperitonealer Injektion des Antigens zur Freisetzung von SRS-A (Smith et al., Int. Arch. Allergy appl. Immunol. 62, 195 1980). Histamin wird hierbei nicht freigesetzt, so daß die lipoxygenasehemmende Wirkung von Prüfsubstanzen selektiv untersucht werden kann.

Die erfindungsgemäßen Verbindungen hemmten die Leukotrienbildung/-freisetzung bei niedrigen Dosierungen (Tabelle 6).

Ergebnisse

A1) Lipoxygenasehemmung im PMN-Leukozyten (Tabelle 1/ Tabelle 2)

Wie die Tabelle 1 zeigt, sind viele Verbindungen bei 5 x $10^{-7}$ g/ml noch mit $>$50 % Hemmung wirksam.

Von den Referenzen ist die NDGA, die therapeutisch nicht verwendbar ist, bei 5 x $10^{-7}$ g/ml mit ca. 50 % wirksam. Das therapeutisch bereits eingesetzte Benoxaprofen ist wesentlich schwächer wirksam ($IC_{50}$ 1 x $10^{-5}$ - 5 x $10^{-5}$ g/ml); (Tabelle 2).

A2) a) Lipoxygenasehemmung in Thrombozyten

Parallel zur Hemmung der 5-Lipoxygenase in Leukozyten wird auch die 12-Lipoxygenase in Thrombozyten gehemmt (Tabelle 1).

b) Cyclooxygenasehemmung in Thrombozyten

Der Cyclooxygenaseweg in Thrombozyten, gemessen über die Bildung von Thromboxan ($TXB_2$) und HHT, wird durch die erfindungsgemäßen Verbindungen erst bei relativ hohen Konzentrationen schwach gehemmt (Tabelle 1).

Dies kann bei bestimmten Indikationen von Vorteil sein, zeigt jedoch andererseits die selektive Wirkung der Benzothiazine.

A3) Prostacyclinstimulation

a) In Mikrosomen (Tabelle 1)

Hemmung des Lipoxygenaseweges verhindert die Bildung von Hydroperoxyfettsäuren (HPETE's), die als Inhibitoren der Prostacyclinsynthetase bekannt sind. Hieraus sollte eine erhöhte Synthese des aggregationshemmenden und gefäßdilatierenden Prostacyclin resultieren.

Wie die Tabelle 1 zeigt, kommt es bei den erfindungsgemäßen Verbindungen zu diesem Effekt.

b) In Aortenringen ex vivo.

Nach Verabreichung der Prüfsubstanz an Ratten wird die Fähigkeit von Aortenringen Prostacyclin zu synthetisieren, erhöht.

Beispiel: Herstellungsbeispiel 7 (30 mg/kg p.o.).

B. Leukozytenklebrigkeit

Die Arachidonsäuremetaboliten der Lipoxygenasewege, insbesondere die Leukotriene, haben eine Vielzahl von wichtigen Mediatorfunktionen: Leukotrien $B_4$ ist stark chemotaktisch und chemokinetisch wirksam, die Leukotriene C und D sind mit der "slow reacting substance of anaphylaxis (SRS-A)" identisch und besitzen lang anhaltende konstriktorische Wirkungen auf die Bronchialmuskulatur sowie auf eine Reihe anderer glatter Muskelzellen und erhöhen die mikrovaskuläre Permeabilität. Für die Mikrozirkulation bedeutet dies, daß die entstehenden Spasmen einmal zur Erhöhung des Widerstandes führen, zum anderen kommt es zu einer Erhöhung der Klebrigkeit von Leukozyten, was zu peripheren Durchblutungsstörungen führt.

Die erfindungsgemäßen Verbindungen zeigten hier eine ausgezeichnete Wirksamkeit (Tabelle 3).

C. Thrombozytenaggregationshemmung (Tabelle 4)

Die Hemmung der Thrombozytenaggregation in vitro und nach Verabreichung an Tiere wird in der Tabelle demonstriert, womit die erfindungsgemäßen Verbindungen zur Prophylaxe und Therapie

von Thrombozytenfunktionsstörungen, Erkrankungen mit Aggregatbildung (TIA, Durchblutungsstörungen, plötzlicher Herztod etc.) und Thrombosen geeignet sind.

D) Thromboembolieprotektion

Die antithrombotische Wirkung der Benzothiazine wird in diesem Thromboemboliemodell gezeigt.

Beispiel

| | Dosis (mg/kg p.o.) | Überlebensrate % |
|---|---|---|
| Kontrolle | - | 0 |
| Herstellungs-beispiel 16 | 100 mg/kg/p.o. | 66,6 |

E) Wirkung auf Reoxygenierungsschäden, demonstriert am nach Langendorff perfundierten Kaninchenherzen

Die Perfusion mit einer Thrombozytensuspension bewirkt unter normoxischen Bedingungen einen signifikanten Anstieg des Perfusionsdruckes (CPP). Dieser Druckanstieg ist nach einer vorangegangenen Hypoxie in der anschließenden Reoxygenierungsphase deutlich verstärkt. 4H-1,4-Benzothiazine inhibieren überraschenderweise den durch Thrombozyten induzierten Perfusionsdruckanstieg. Dieser inhibierende Effekt ist in der Reoxygenierungsphase deutlich verstärkt.

F) Koronarthrombose

4H-1,4-Benzothiazine inhibieren die experimentelle Koronarthrombose.

G) Herzinfarkt und Herzrhytmusstörungen

Nach der graduellen, langsamen LAD-Unterbindung verkleinern 4H-1,4-Benzothiazine den Infarkt. Dies war zu beobachten sowohl in Bezug auf das absolute Infarktgewicht als auch in Bezug auf die Infarktgröße relativ zum linken Ventrikelgewicht. Initial sank als Folge der Koronarligatur der Blutdruck, war zur refelktorischen Herzfrequenzsteigerung führte. Sonst wurden keine durch 4H-1,4-Benzothiazin verursachten hämodynamischen Wirkungen gesehen. Die Verbindungen verminderten die infarktbedingten Erhöhungen der St-Segmente unter der R-Zacke des peripheren EKG als Ischämiezeichen.

H) Erkrankungen des Respirationstraktes (Tabelle 5, 6)

1. Wichtigstes Zielorgan für Allergien ist die Lunge. Es kommt zu einer selektiven

Hemmung der Lipoxygenase in Homogenaten menschlicher Lungen.

2. Die Freisetzung von Mediatorsubstanzen aus menschlichem Lungengewebe wird ebenfalls bei niedrigen Konzentrationen gehemmt.

3./4. Die hervorragende antiasthmatische Wirkung der erfindungsgemäßen Verbindungen wurde auch in zwei in vivo Modellen gezeigt. Die durch Antigen erzeugten Bronchokonstriktionen in sensibilisierten Meerschweinchen werden signifikant reduziert. Die Freisetzung von Leukotrienen bei einer passiven, peritonealen Anaphylaxie (Ratte) wird ebenfalls gehemmt.

Tabelle 1 Wirkung von Benzothiazinderivaten auf den Arachidonsäuremetabolismus

- = nicht aktiv
+ = schwach aktiv
++ = aktiv

| Herstellungs-beispiel Nr. | | Konz. /μg/ml/ | Lipoxygenase-Weg: Hemmung der $LTB_4$-Synthese in menschlichen polymorphkernigen Leukozyten | Lipoxygenase-Weg: Hemmung der 12-HETE-Synth. in Thrombozyten | Cyclooxygenase-Weg: Hemmung der $TXB_2$/HHT-Synth. in Thrombozyten | Cyclooxygenase-Weg: Stimulation der $PGI_2$-Synth. in Mikrosomen |
|---|---|---|---|---|---|---|
| 12 | 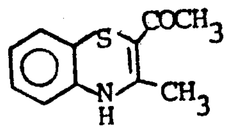 | 10 | 100 | | | |
| | | 5 | 100 | | | |
| | | 1 | 40 | | | |
| 13 | 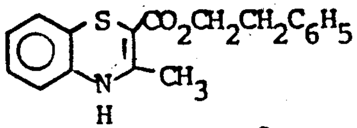 | 10 | 100 | | | |
| | | 5 | 100 | | | |
| | | 1 | 60 | | | |
| 25 | 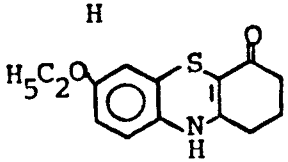 | 100 | | | | ++ |
| | | 10 | 100 | | | ++ |
| | | 5 | 81 | | | |
| | | 1 | 66 | | | - |
| 29 | 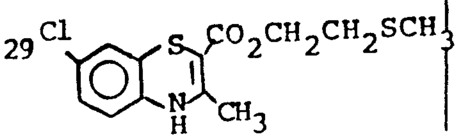 | 10 | 100 | ++ | ++ | - |
| | | 5 | 100 | | | |
| | | 1 | 83 | ++ | + | |
| | | 0,5 | 66 | | | |
| 32 | 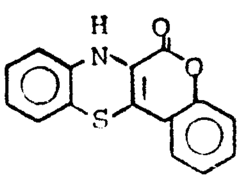 | 100 | - | | | ++ |
| | | 10 | - | ++ | + | + |
| | | 1 | - | + | + | |

Tabelle 1 (Fortsetzung)

| Herstellungs-beispiel Nr. | | Konz. [µg/ml] | Lipoxygenase-Weg: Hemmung der $LTB_4$-Synthese in menschlichen polymorphkernigen Leukozyten | Lipoxygenase-Weg: Hemmung der 12-HETE-Synth. in Thrombozyten | Cyclooxygenase-Weg: Hemmung der $TXB_2$/HHT-Synth. in Thrombozyten | Cyclooxygenase-Weg: Stimulation der $PGI_2$-Synth. in Mikrosomen |
|---|---|---|---|---|---|---|
| 33 | 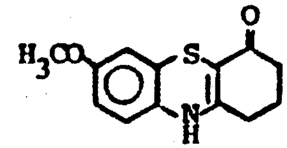 | 100 | | | | ++ |
| | | 10 | 100 | ++ | + | ++ |
| | | 5 | 84 | | | |
| | | 1 | 34 | - | - | - |
| 34 | 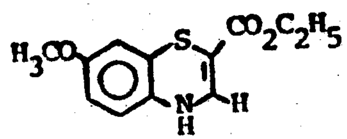 | 10 | 100 | ++ | ++ | - |
| | | 5 | 97 | | | |
| | | 1 | 82 | ++ | + | |
| | | 0,5 | 60 | | | |
| 35 | 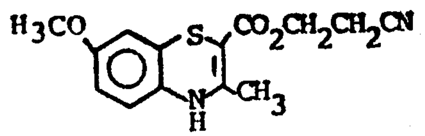 | 100 | | | | ++ |
| | | 10 | 100 | | | + |
| | | 5 | 89 | | | |
| | | 1 | 52 | | | - |
| 36 | 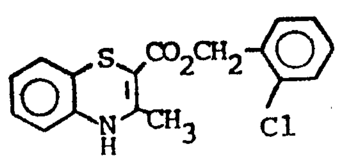 | 100 | - | | | + |
| | | 10 | 100 | | | - |
| | | 5 | 100 | | | |
| | | 1 | 100 | | | - |
| | | 0,5 | 78 | | | |

Tabelle 1 (Fortsetzung)

| Herstellungs- beispiel Nr. | | Konz. /µg/ml/ | Lipoxygenase-Weg: Hemmung der $LTB_4$-Synthese in menschlichen polymorphkernigen Leukozyten | Lipoxygenase-Weg: Hemmung der 12-HETE-Synth. in Thrombozyten | Cyclooxygenase-Weg: Hemmung der $TXB_2$/HHT-Synth. in Thrombozyten | Cyclooxygenase-Weg: Stimulation der $PGI_2$-Synth. in Mikrosomen |
|---|---|---|---|---|---|---|
| 40 | 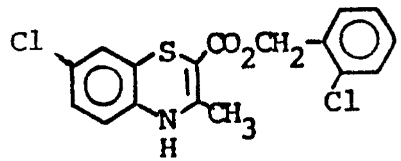 | 10 | 100 | | | |
| | | 5 | 94 | | | |
| 41 | 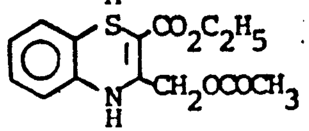 | 10 | 100 | | | |
| | | 5 | 87 | | | |
| | | 1 | 41 | | | |
| 45 | 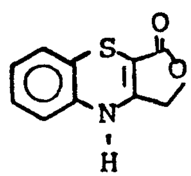 | 10 | 100 | | | |
| | | 5 | 100 | | | |
| | | 1 | 28 | | | |
| 50 | 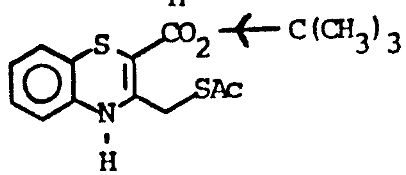 | 10 | 100 | | | |
| | | 5 | 82 | | | |
| | | 1 | 14 | | | |
| 54 | 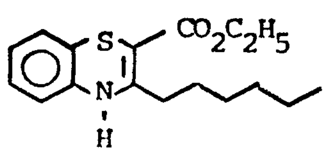 | 10 | 100 | | | |
| | | 5 | 100 | | | |
| | | 1 | 89 | | | |
| | | 0,5 | 41 | | | |

Tabelle 1 (Fortsetzung)

| Herstellungs-beispiel Nr. | | Konz. /μg/ml/ | Lipoxygenase-Weg: Hemmung der $LTB_4$-Synthese in menschlichen polymorphkernigen Leukozyten | Lipoxygenase-Weg: Hemmung der 12-HETE-Synth. in Thrombozyten | Cyclooxygenase-Weg: Hemmung der $TXB_2$/HHT-Synth. in Thrombozyten | Cyclooxygenase-Weg: Stimulation der $PGI_2$-Synth. in Mikrosomen |
|---|---|---|---|---|---|---|
| 58 | 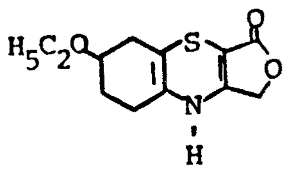 | 10 | 95 | | | |
| | | 5 | 91 | | | |
| | | 1 | 37 | | | |
| | | 0,5 | 6 | | | |
| 61 | 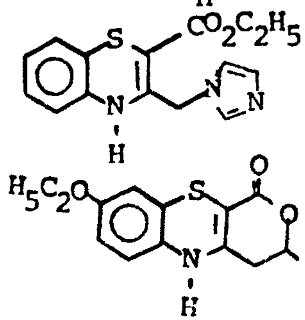 | 10 | 71 | | | |
| | | 5 | 57 | | | |
| | | 1 | 11 | | | |
| 70 | | 10 | 100 | | | |
| 72 | 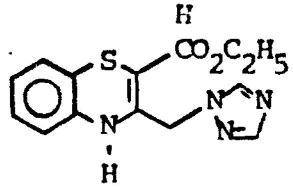 | 10 | 89 | | | |
| | | 5 | 55 | | | |
| | | 1 | 3 | | | |

Tabelle 1 (Fortsetzung)

| Herstellungs-beispiel Nr. | | Konz. /µg/ml/ | Lipoxygenase-Weg: Hemmung der $LTB_4$-Synthese in menschlichen polymorphkernigen Leukozyten | Lipoxygenase-Weg: Hemmung der 12-HETE-Synth. in Thrombozyten | Cyclooxygenase-Weg: Hemmung der $TXB_2$/HHT-Synth. in Thrombozyten | Cyclooxygenase-Weg: Stimulation der $PGI_2$-Synth. in Mikrosomen |
|---|---|---|---|---|---|---|
| 73 | 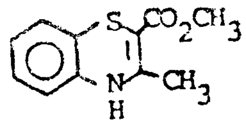 | 10 | 100 | | | |
| | | 5 | 100 | | | |
| | | 1 | 90 | | | |
| | | 0,5 | 65 | | | |
| 84 | 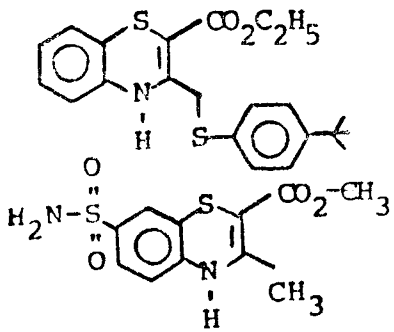 | 10 | 90 | | | |
| | | 5 | 85 | | | |
| | | 1 | 39 | | | |
| 94 | | 10 | 47 | | | |
| | | 5 | 16 | | | |
| 104 | 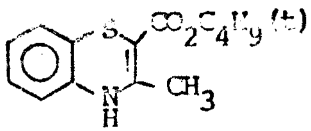 | 10 | 100 | | | |
| | | 5 | 100 | | | |
| | | 1 | 64 | | | |

Tabelle 1 (Fortsetzung)

| Herstellungs- beispiel Nr. | | Konz. [µg/ml] | Lipoxygenase-Weg: Hemmung der $LTB_4$-Synthese in menschlichen polymorphkernigen Leukozyten | Lipoxygenase-Weg: Hemmung der 12-HETE-Synth. in Thrombozyten | Cyclooxygenase-Weg: Hemmung der $TXB_2$/HHT-Synth. in Thrombozyten | Cyclooxygenase-Weg: Stimulation der $PGI_2$-Synth. in Mikrosomen |
|---|---|---|---|---|---|---|
| 107 | 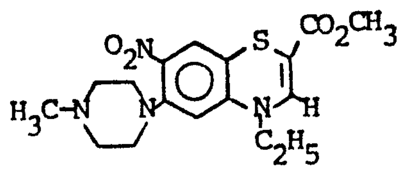 | 10 | 53 | | | |
| 108 | 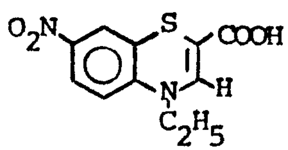 | 10 | 66 | | | |

Tabelle 2 (Vergleich zur Tabelle 1)

Hemmung der $LTB_4$ Freisetzung aus menschlichen PMN-Leukozyten

| | $IC_{50}$ | |
|---|---|---|
| Verbindung | µg/ml | µM |
| Phenidon | 1 | 6 |
| BW 755 C | 5-10 | 22-44 |
| NDGA | 0.5 | 1.6 |
| Benoxaprofen | 10-50 | 33-66 |

Tabelle 3

Adhäsion von Leukozyten in Venulen (30-40 µm)

| Herstellungs-beispiel | Dosis (mg/kg) | Applikations-form | Erhöhung der Anzahl der Leukozyten pro Zeiteinheit in % | Wirkdauer (min) |
|---|---|---|---|---|
| 16 | 3 | i.a. (fem.) | 110 | > 60 |
| 67 | 1 | i.a. (fem.) | 220 | > 60 |
| 71 | 1 | i.a. (fem.) | 90 | > 60 |
| 106 | 1 | i.a. (fem.) | 180 | > 60 |

Tabelle 4

Hemmung der Thrombozytenaggregation

| Substanz (Nummer des Herstellungsbeispiels) | in vitro Grenzkonzentrationen für Hemmung (µg/ml) | ex vivo Dosis (mg/kg p.o.) | Effekt (%) |
|---|---|---|---|
| 1 | 10-3 | 100 | 0 |
| 7 | 10-3 | 100 | ~50 |
| 16 | 3-1 | 100 | >50 |
| | | 30 | <50 |
| 26 | 3-1 | 100 | 0 |
| 27 | 3-1 | 100 | >50 |
| | | 30 | >50 |
| 28 | 10-3 | 100 | >50 |
| | | 30 | ~50 |

Tabelle 5 Anti-asthmatisches Profil von Benzothiazinen: In vitro Tests

| Verbindung (Herstellungs-beispiel) | Lungenhomogenat (human) % Hemmung (5 µg/ml) 5-LO | CO | Mediator-Freisetzung in menschlichen Lungen % Hemmung (10µg/ml) SRS-A | $TXB_2$ | Histamin |
|---|---|---|---|---|---|
| 16 | 73 | 6 | 82 | 73 | 14 |
| 1(6) | 91 | 15 | 71 | 100 | (7) |
| 7 | 94 | 75 | 28 | 95 | (12) |
| 9 | 95 | 74 | 15 | (55) | (13) |
| BW 755C | nt | nt | 100 | 100 | (10) |
| INDOMETACIN | 18 | 66 | | | |
| PHENIDON | 71 | 24 | 100 | 100 | 4 |

nt = nicht geprüft

LO = Lipoxygenase

CO = Cyclooxygenase

( ) = gesteigerte Freisetzung/Stimulation

Tabelle 6 Antiasthmatisches Profil von Benzothiazinen: In vivo Tests

| Verbindung (Herstellungs-beispiel) | Konzett-Rössler (Meerschweinchen) % Hemmung (Max. Effekt) | | Passive peritoneale Anaphylaxie (Ratte) % Hemmung (10 mg $kg^{-1}$) | |
|---|---|---|---|---|
| | 5 min nach Inhalation der Substanz | 30 min nach oraler Gabe | Intraperitioneal (30 min) | Oral (60 min) |
| 16 | 32 % * (0,5 mg) | 27 % (1 mg $kg^{-1}$) | 26 % | nt |
| 1(6) | 38 % (0,5 & 1 mg) | 43 % (1 mg $kg^{-1}$) | 18 % | 22 % |
| 7 | 55 % ** (1 mg) | 49 ** (3 mg $kg^{-1}$) | 80 % | nt |
| 9 | 34 % ** (1 mg) | 57 % (1 mg $kg^{-1}$) | 39 % | nt |
| INDOMETACIN | nt | nt | nt | + 31 % (3 mg kg-1) |
| BW 755 C | 27 % * (0,5 mg) | 23 % (10 mg $kg^{-1}$) | 12 % | 49 % |
| PHENIDON | 38 % (0,5 mg) | 62 % * 0,3 mg $kg^{-1}$) | 67 % | 52 % |

* $p < 0,05$    ** $p < 0.01$    nt = nicht geprüft    + = gesteigerte Freisetzung

## Herstellungsbeispiele

### Beispiel 1

3-Methyl-4H-1,4-benzothiazin-2-carbonsäure-(2-cyanethyl)-ester

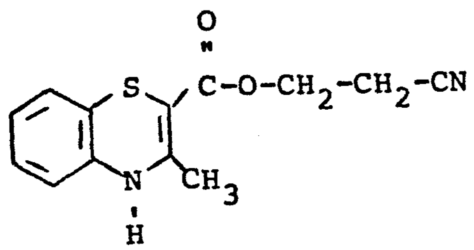

10 g (0,08 Mol) 2-Aminothiophenol und 12,4 g (0,08 Mol) Acetessigsäure-(2-cyanethyl)-ester wurden in 40 ml Dimethylsulfoxid gelöst. Das Gemisch wurde 2 h bei 140°C gehalten und nach dem Erkalten mit 250 ml Wasser versetzt. Es bildete sich ein Niederschlag, der abgesaugt und aus Isopropanol umkristallisiert wurde.

Fp. 154°C

Ausbeute: 8,2 g (39 % der Theorie).

Beispiel 2

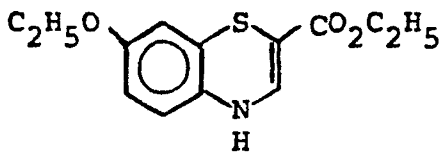

5,5'-Diethoxy-2,2'-diaminodiphenyldisulfid wurden 100,8 g (0,3 mol) bei Raumtemperatur in 150 ml DMSO gelöst und auf 120°C erhitzt. Zwischen 120-130°C wurde in ca. 30 min 60,7 ml (0,6 mol) Propiolsäureester zugetropft und weitere 3 Stunden bei 120°C gerührt (DC in 7 $Et_2O$/3Hex, anschließend mit Ninhydrin besprühen). Nach Abkühlen auf 40°C wurde die Reaktionsmischung unter schnellem Rühren in eine Vorlage aus 1500 ml $H_2O$ und 500 g Eis sehr langsam ausgetragen.

Nach 30-45 min Rühren, kristallisierte das Rohprodukt grobkörnig. Nach Absaugen wurde 2 mal mit 500 ml Wasser gewaschen.

Das Rohprodukt wurde im Hochvakuum mit Trockenmittel Siccapent gut getrocknet. Rohausbeute: 148,1 g. Das getrocknete Rohprodukt wurde 30 min in 1,5 l $Et_2O$/RT verrührt und erneut gut abgesaugt. Mit wenig $ET_2O$ wurde nachgewaschen.

Das scharf abgesaugte Produkt wurde nachgewaschen, in 2,5 l MeOH eingetragen, mit 25 g A-Kohle und 25 g Kieselgel 00,63-0200 versetzt und unter Stickstoff und unter Rühren 30 min am Rückfluß gekocht. Anschließend wurde heiß über einen beheizbaren Filter

mit Doppel-Papierfilter abfiltriert und über Nacht abgekühlt. Das Produkt kristallisierte in gelben feinen Nadeln aus. Nach Absaugen wurde mit wenig MeOH gewaschen und im Hochvakuum getrocknet.

Ausbeute: 32,2 g (20,2 %), Fp: 171°C.

Das Filtrat wird einrotiert und erneut aus 300 ml MeOH mit A-Kohle und Kieselgel umkristallisiert. Absaugen, trocknen. Ausbeute: 4,8 g (3,0 %), Fp: 171°C.

Beispiel 3

3-Benzyl-4H-1,4-benzothiazin-2-carbonsäure-ethylester

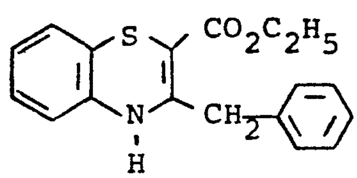

Zu einer siedenden Lösung von 24,8 g (0,1 Mol) Dithio-2,2-dianilin und 100 mg p-Toluolsulfonsäure in 300 ml Toluol wurde unter Stickstoff eine Lösung von 20,6 g (0,1 Mol) 4-Phenylacetessigsäureethylester in 100 ml Toluol getropft. Das Gemisch wurde 5 Stunden am Wasserabscheider gekocht. Nach dem Erkalten wurde mit Sodalösung gewaschen, die organische Phase abgetrennt, mit $Na_2SO_4$ getrocknet und eingeengt.

Der Rückstand wurde chromatographisch (Kieselgel 0,063 - 0,2) aufgetrennt. Es wurden 4,8 g vom Fp. 140-143°C erhalten.

<u>Beispiel 4</u>

7-Ethoxy-1-oxo-1,2,3,4-tetrahydrophenothiazin

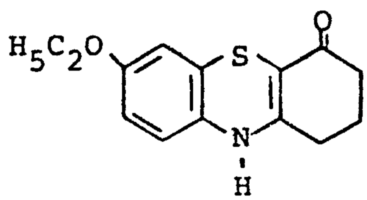

Eine Lösung von 5,4 g (0,016 Mol) 4,4'-Diethoxy-2,2'-dithiodianilin und 3,6 g (0,032 Mol) 1,3-Cyclohexandion in 20 ml Dimethylsulfoxid wurde eine Stunde auf 110-120°C erhitzt. Nach dem Erkalten wurde das Gemisch auf 100 ml Wasser gegeben und mit Aceton versetzt. Es bildete sich ein Niederschlag, der abgesaugt und aus DMF/Isopropanol umkristallisiert wurde.

Fp. 201-202°C
Ausbeute 5 g (59 % der Theorie).

In gleicher Weise lassen sich folgende Präparate herstellen:

| Beispiel Nr. | Strukturformel | Fp. |
|---|---|---|
| 5 | 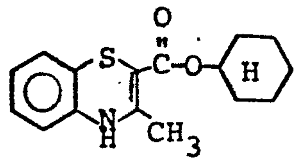 | 161°C |
| 6 | 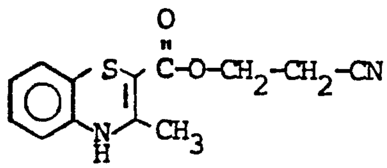 | 154°C |
| 7 | 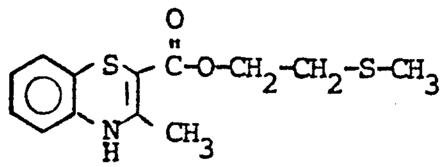 | 132°C |
| 8 | 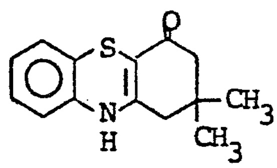 | 270°C |
| 9 | 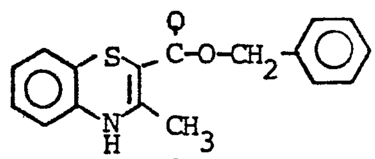 | 155°C |
| 10 | 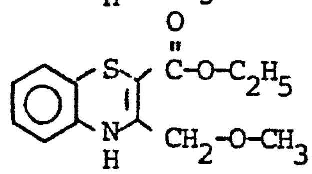 | 179–183°C |
| 11 | 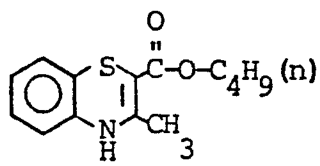 | 86°C |
| 12 | 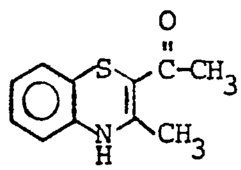 | 196°C |

| Beispiel Nr. | Strukturformel | Fp. |
|---|---|---|
| 13 | 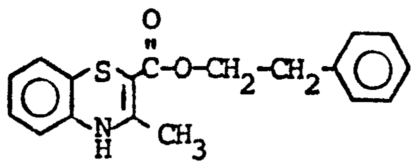 | 136°C |
| 14 | 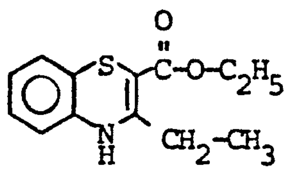 | 122°C |
| 15 | 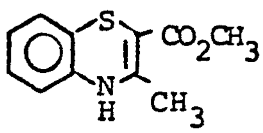 | 150°C |
| 16 | 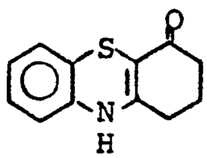 | 153°C |
| 17 | 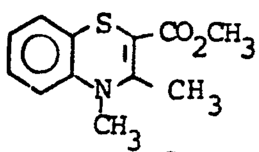 | 118°C |
| 18 | 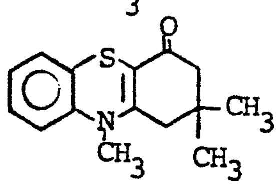 | 153°C |
| 19 | 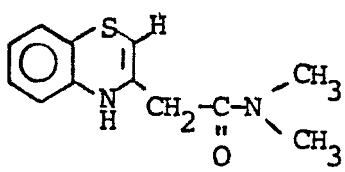 | 163°C |
| 20 | 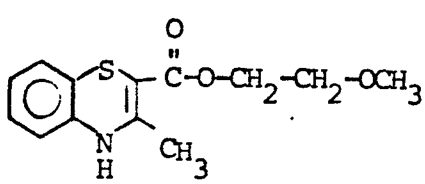 | 108°C |

| Beispiel Nr. | Strukturformel | Fp. |
|---|---|---|
| 21 | 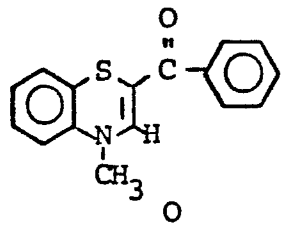 | 161°C |
| 22 | 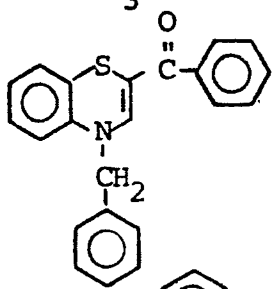 | 171°C |
| 23 | 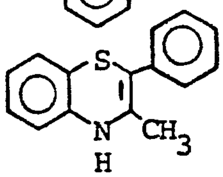 | Öl |
| 24 | 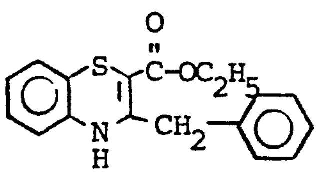 | 143°C |
| 25 | 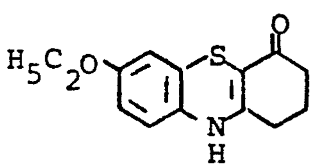 | 202°C |
| 26 | 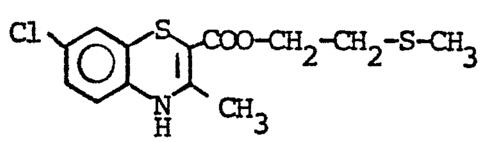 | 123°C |
| 27 | 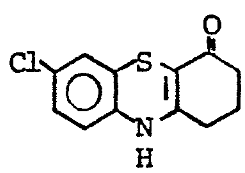 | 250°C |

| Beispiel Nr. | Strukturformel | Fp. |
|---|---|---|
| 28 | 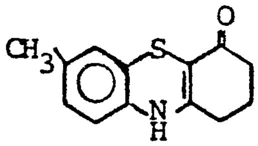 | 231°C |
| 29 | 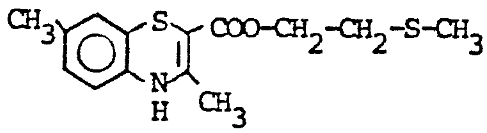 | 152-154°C |
| 30 | 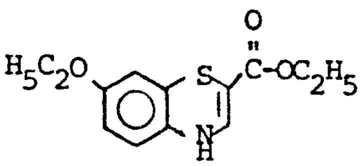 | 171°C |
| 31 | 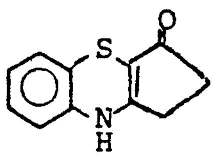 | >300°C |
| 32 | 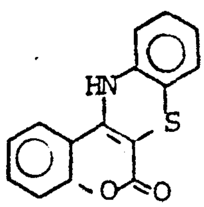 | >240°C |
| 33 | 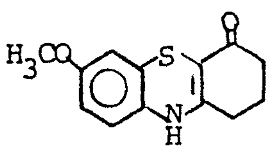 | 213-213°C |
| 34 | 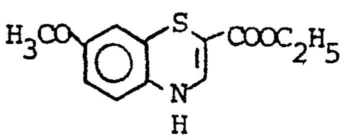 | 132-133°C |
| 35 | 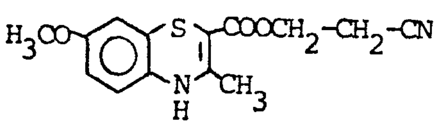 | 167-168°C |

| Beispiel Nr. | Strukturformel | Fp. |
|---|---|---|
| 36 | 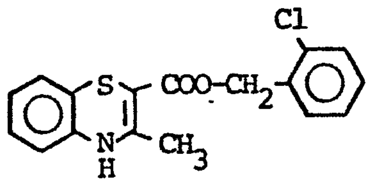 | 161-162°C |
| 37 | 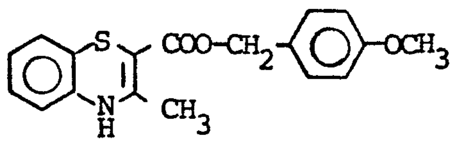 | 131-133°C |
| 38 | 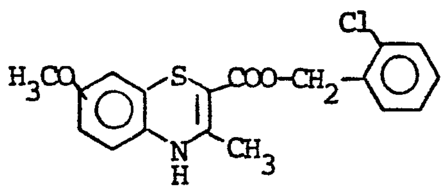 | 145-146°C |
| 39 | 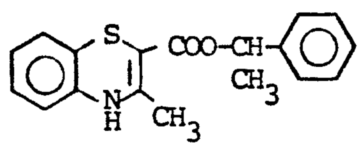 | 114-115°C |
| 40 | 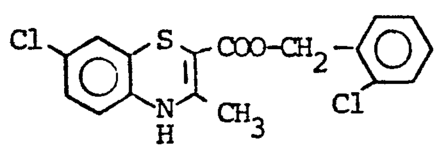 | 178-180°C |
| 41 | 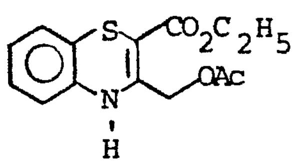 | 165-168°C |
| 42 | 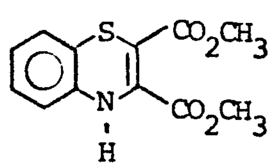 | 107°C |
| 43 | 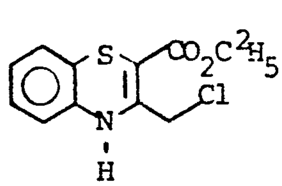 | 125°C |

| Beispiel Nr. | Strukturformel | Fp. |
|---|---|---|
| 44 | 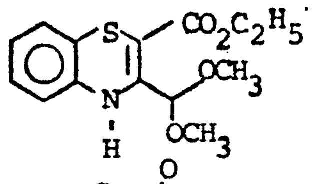 | Öl |
| 45 | 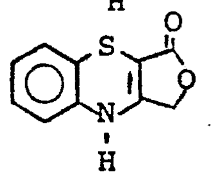 | 248°C |
| 46 | 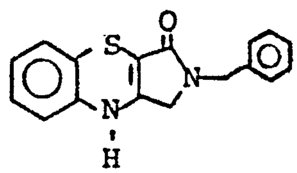 | 187°C |
| 47 | 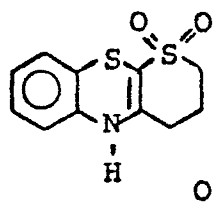 | 268°C |
| 48 | 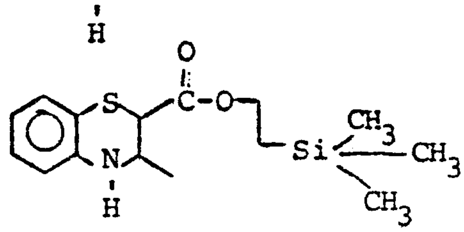 | 113°C |
| 49 | 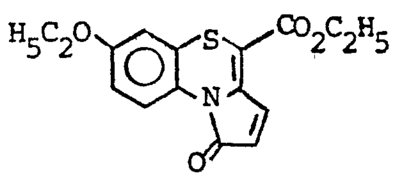 | 188°C |
| 50 | 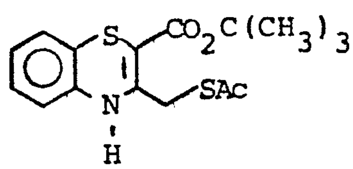 | 138°C |
| 51 | 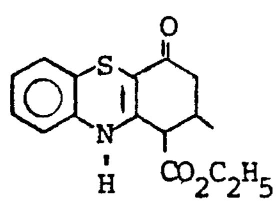 | 203°C |

| Beispiel Nr. | Strukturformel | Fp. |
|---|---|---|
| 52 | 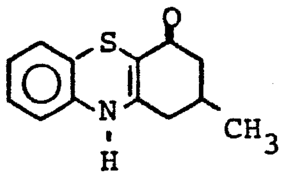 | 269-270°C |
| 53 | 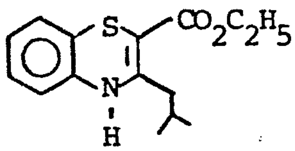 | 104°C |
| 54 | 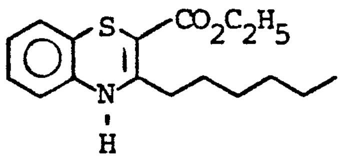 | 98°C |
| 55 | 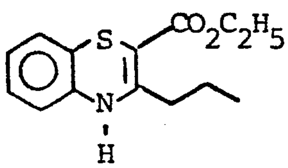 | 118°C |
| 56 | 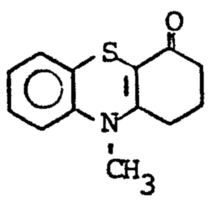 | 156°C |
| 57 | 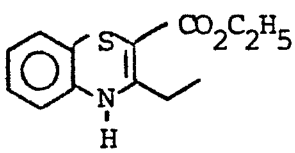 | 120°C |
| 58 | 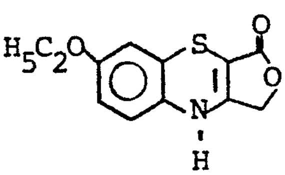 | 263°C |

| Beispiel Nr. | Strukturformel | Fp. |
|---|---|---|
| 59 | 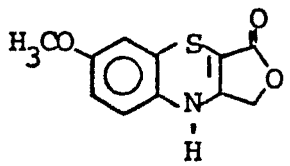 | 281°C |
| 60 | 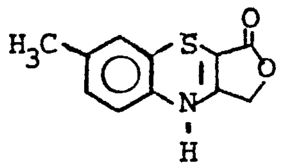 | 302°C |
| 61 | 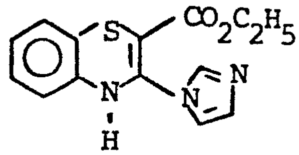 | 183-186°C |
| 62 | 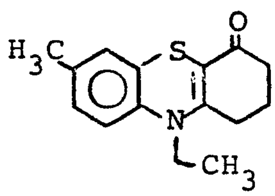 | 127°C |
| 63 | 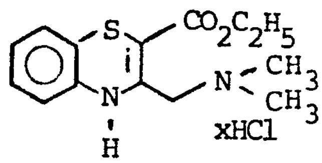 | 78°C |
| 64 | 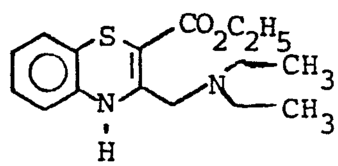 | Öl |
| 65 | 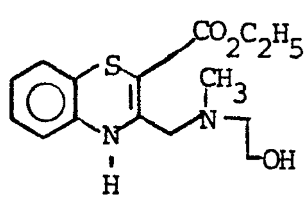 | 143°C |

| Beispiel Nr. | Strukturformel | Fp. |
|---|---|---|
| 66 | 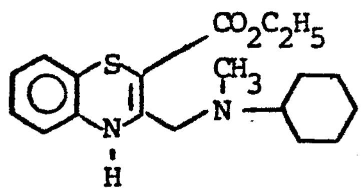 | 56°C |
| 67 | 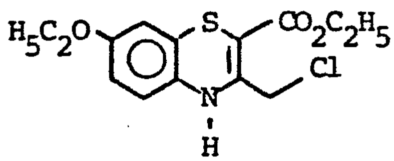 | 118°C |
| 68 | 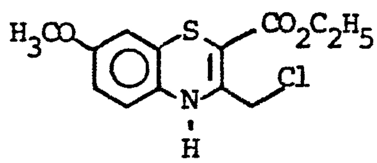 | 123°C |
| 69 | 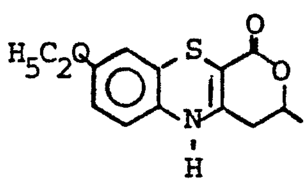 | 227°C |
| 70 | 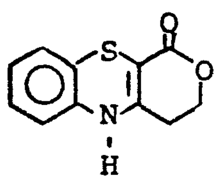 | 222°C |
| 71 | 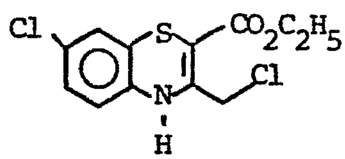 | 118°C |
| 72 | 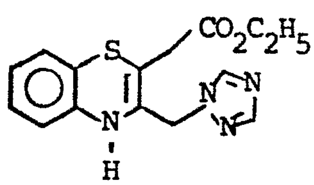 | 165°C |
| 73 | 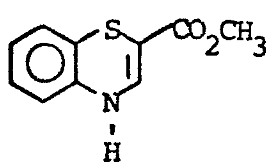 | 148-149°C |

| Beispiel Nr. | Strukturformel | Fp. |
|---|---|---|
| 74 | 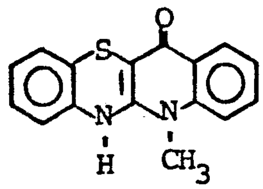 | 253°C |
| 75 | 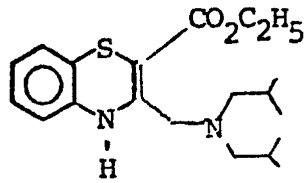 | 137°C |
| 76 | 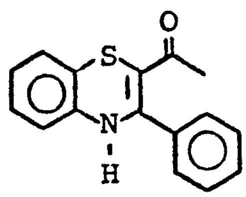 | 184-18[illegible]°C |
| 77 | 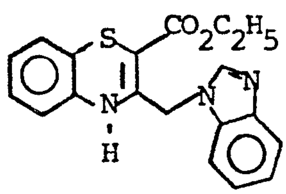 | 197°C |
| 78 | 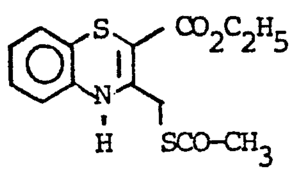 | 89-91°C |
| 79 | 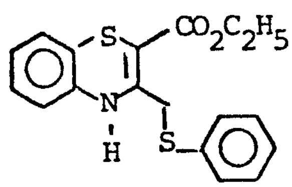 | 91-92°C |

| Beispiel Nr. | Strukturformel | Fp. |
| --- | --- | --- |
| 80 | 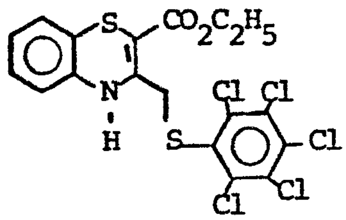 | 78-80°C |
| 81 | 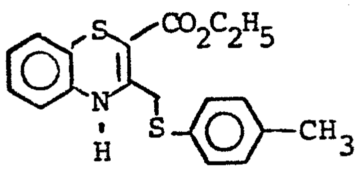 | Öl |
| 82 | 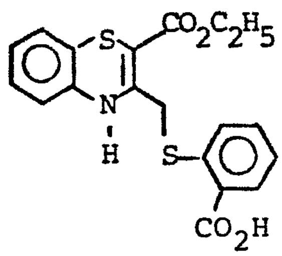 | 178-179°C |
| 83 | 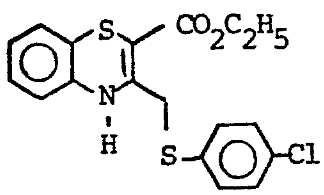 | Öl |
| 84 | 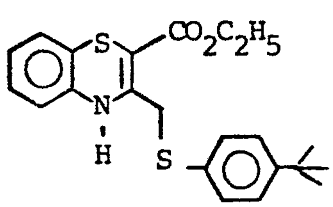 | Öl |

| Beispiel Nr. | Strukturformel | Fp. |
|---|---|---|
| 85 | 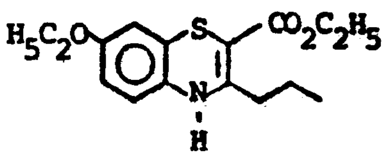 | 85°C |
| 86 | 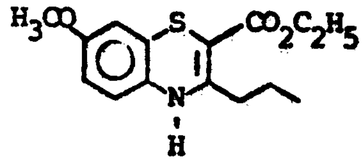 | 74°C |
| 87 | 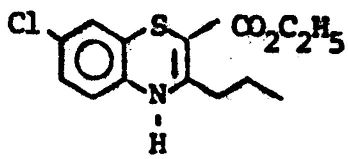 | 120°C |
| 88 | 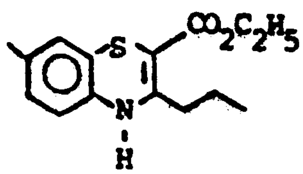 | 104°C |
| 89 | 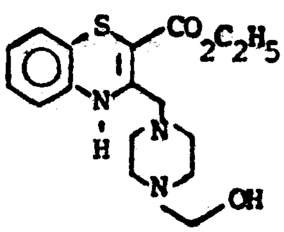 | 96-8°C |
| 90 | 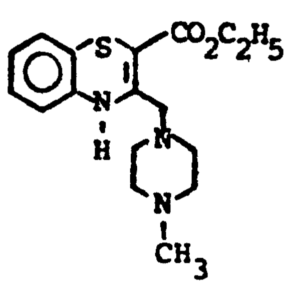 | 87°C |

| Beispiel Nr. | Strukturformel | Fp. |
|---|---|---|
| 91 | 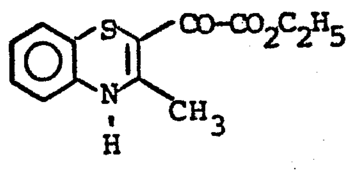 | 145°C |
| 92 | 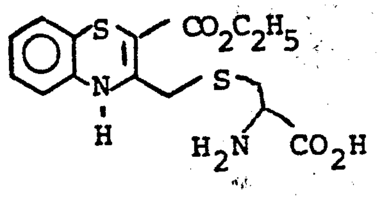 x $H_2O$ | 175°C Z |
| 93 | 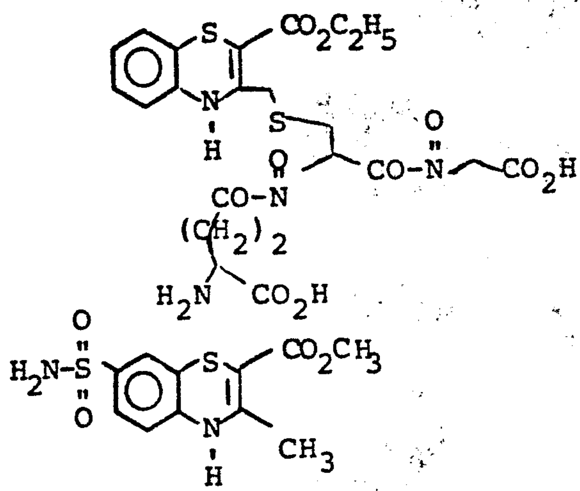 | 199–203°C Z |
| 94 | | 253°C Z |
| 95 | 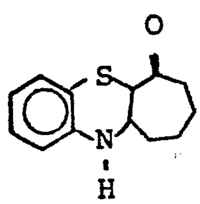 | 240–242°C Z |
| 96 | 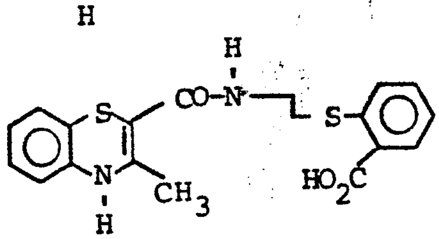 | 157–159°C |

| Beispiel Nr. | Strukturformel | Fp. |
|---|---|---|
| 97 | 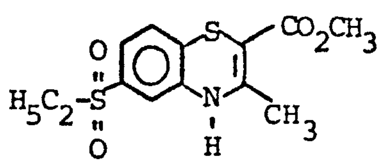 | 163-168°C |
| 98 | 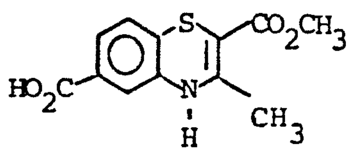 | 130°C Z |
| 99 | 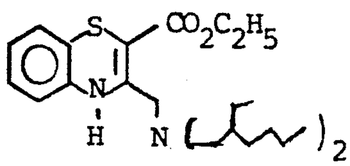 | Öl |
| 100 | 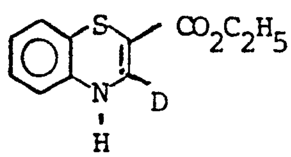 | 171-174°C |
| 101 | 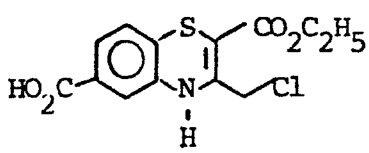 | 300°C |
| 102 | 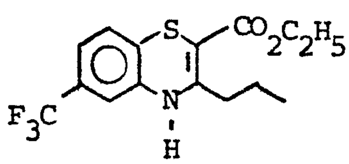 | 111-115°C |
| 103 | 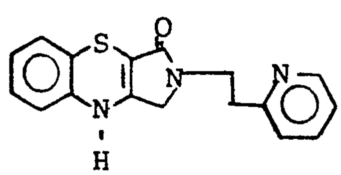 | 210°C Z |

| Beispiel Nr. | Strukturformel | Fp. |
|---|---|---|
| 104 | 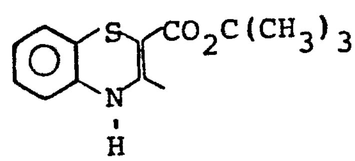 | 157°C |
| 105 | 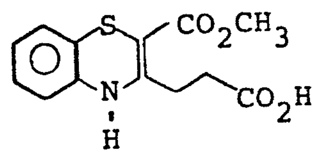 | 149°C |
| 106 | 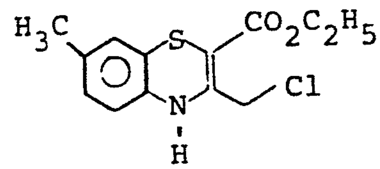 | 120°C |
| 107 | 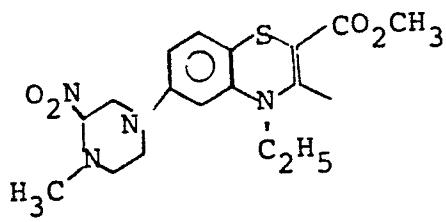 | 156-9°C |
| 108 | 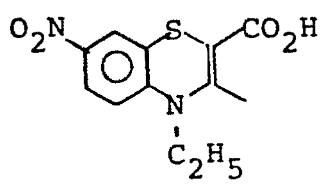 | 193-5°C |

Patentansprüche:

1. Verwendung von 4H-1,4-Benzothiazinen der allgemeinen Formel I

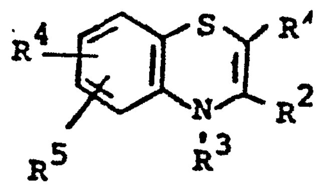

(I)

worin

$R^1$ für Wasserstoff, Aryl (gegebenenfalls substituiertes), Hetaryl (gegebenenfalls substituiertes), Alkyl (gegebenenfalls substituiertes), Cyano, $-CO-R^6$, wobei $R^6$ und $R^2$ durch Brückenglieder verbunden sein können,

$R^2$ für Wasserstoff, Deuterium, Alkyl (gegebenenfalls substituiertes), Aryl (gegebenenfalls substituiertes), $-CH_2-R^6$, $-CO-R^6$, wobei $R^6$ und $R^1$ durch Brückenglieder verbunden sein können,

$R^6$ und $R^2$ bzw. $R^6$ und $R^1$ für Alkylen mit 2 bis 4 C-Atomen steht, das ein- oder mehrfach substituiert sein kann durch Alkyl, Alkoxy, Aryl, Fluor, Alkoxycarbonyl, wobei eine Methylengruppe auch durch O, S, SO, $SO_2$, NH oder N-Alkyl ersetzt sein kann,

$R^3$ für Wasserstoff, Alkyl (gegebenenfalls substituiertes), Acyl,

$R^4$, $R^5$ für Wasserstoff, Alkyl (gegebenenfalls substituiertes, z.B. Trifluormethyl), Halogen, Alkoxy, Nitro, Acyloxy, Cyano, N-Alkylpiperazino, Alkylsulfonyl, Arylsulfonyl, Amidosulfonyl, Carboxyl und wobei $R^4$ und $R^5$ gleich oder verschieden sein können,

$R^3$ für Wasserstoff, Alkyl (gegebenenfalls substituiertes),

$R^4$, $R^5$ für Wasserstoff, Alkyl (gegebenenfalls substituiertes), Halogen, Alkoxy, Nitro, Acyloxy, Cyano, Alkylpiperazino, Alkylsulfonyl, Arylsulfonyl, Amidosulfonyl, Carboxyl, und wobei $R^4$ und $R^5$ gleich oder verschieden sein können, und

$R^6$ für Wasserstoff, Alkyl (gegebenenfalls substituiertes), Aryl (gegebenenfalls substituiertes), Cycloalkyl, Amino (gegebenenfalls substituiertes), gegebenenfalls substituiertes Alkoxy, Cycloalkoxy, Alkoxycarbonyl, gegebenenfalls substituiertes Thioalkyl und Thiophenyl steht,

X für CH oder N

steht, zur Prophylaxe und Therapie von Erkrankungen der Atemwege, Entzündungen, Rheuma, thromboembolischen Erkrankungen, Ischämien und Infarkten, Herzrhythmusstörungen, Arteriosklerosen und Dermatosen.

2. Antiischämisches und antiarrhythmisches Arzneimittel, dadurch gekennzeichnet, daß es eine therapeutisch wirksame Menge einer Verbindungen gemäß Formel (I)

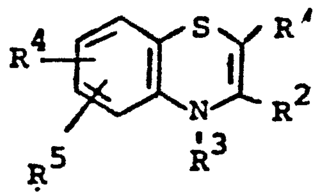

(I)

worin

$R^1$ für Wasserstoff, Aryl (gegebenenfalls substituiertes), Hetaryl (gegebenenfalls substituiertes), Alkyl (gegebenenfalls substituiertes), Cyano, $-CO-R^6$, wobei $R^6$ und $R^2$ durch Brückenglieder verbunden sein können,

$R^2$ für Wasserstoff, Deuterium, Alkyl (gegebenenfalls substituiertes), Aryl (gegebenenfalls substituiertes), $-CH_2-R^6$, $-CO-R^6$, wobei $R^6$ und $R^1$ durch Brückenglieder verbunden sein können,

$R^6$ und $R^2$ bzw. $R^6$ und $R^1$ für Alkylen mit 2 bis 4 C-Atomen steht, das ein- oder mehrfach substituiert sein kann durch Alkyl, Alkoxy, Aryl, Fluor, Alkoxycarbonyl,

wobei eine Methylengruppe auch durch O, S, SO, $SO_2$, NH oder N-Alkyl ersetzt sein kann,

$R^3$ für Wasserstoff, Alkyl (gegebenenfalls substituiertes), Acyl,

$R^4$, $R^5$ für Wasserstoff, Alkyl (gegebenenfalls substituiertes, z.B. Trifluormethyl), Halogen, Alkoxy, Nitro, Acyloxy, Cyano, N-Alkylpiperazino, Alkylsulfonyl, Arylsulfonyl, Amidosulfonyl, Carboxyl und wobei $R^4$ und $R^5$ gleich oder verschieden sein können, und

$R^3$ für Wasserstoff, Alkyl (gegebenenfalls substituiertes),

$R^4$, $R^5$ für Wasserstoff, Alkyl (gegebenenfalls substituiertes), Halogen, Alkoxy, Nitro, Acyloxy, Cyano, Alkylpiperazino, Alkylsulfonyl, Arylsulfonyl, Amidosulfonyl, Carboxyl, und wobei $R^4$ und $R^5$ gleich oder verschieden sein können, und

$R^6$ für Wasserstoff, Alkyl (gegebenenfalls substituiertes), Aryl (gegebenenfalls substituiertes) Cycloalkyl, Amino (gegebenenfalls substituiertes), gegebenenfalls substituiertes Alkoxy, Cycloalkoxy, Alkoxycarbonyl,

gegebenenfalls substituiertes Thioalkyl und Thiophenyl steht,

X für CH oder N steht,

enthält.

3. Arzneimittel zur Verhütung und Behandlung von Erkrankungen der Atenwege, dadurch gekennzeichnet, daß es eine therapeutisch wirksame Menge einer Verbindung gemäß Formel (I) in Anspruch 2 enthält.

4. Arzneimittel zur Verhütung und Bekämpfung von Dermatosen, dadurch gekennzeichnet, daß es eine therapeutisch wirksame Menge einer Verbindung gemäß Formel (I) in Anspruch 2 enthält.

5. Arzneimittel zur Verhütung und Bekämpfung von Entzündungen, dadurch gekennzeichnet, daß es eine therapeutisch wirksame Menge einer Verbindung gemäß Formel (I) in Anspruch 2 enthält.

6. Arzneimittel zur Verhützung und Bekämpfung von Arteriosklerose, dadurch gekennzeichnet, daß es eine therapeutisch wirksame Menge einer Verbindung gemäß Formel (I) in Anspruch 2 enthält.

7. Arzneimittel nach den Ansprüchen 2 bis 6, dadurch gekennzeichnet, daß es eine für die orale Verabreichung geeignete Form aufweist.

8. Arzneimittel nach den Ansprüchen 2 bis 6, dadurch gekennzeichnet, daß es eine für die parenterale Verabreichung geeignete Form aufweist.

9. Verfahren zur Herstellung eines Arzneimittels nach den Ansprüchen 2 bis 8, dadurch gekennzeichnet, daß man eine therapeutisch wirksame Menge einer Verbindung gemäß Formel (I)

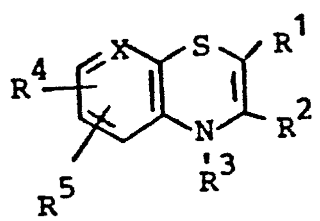

(I)

worin

$R^1$ für Wasserstoff, Aryl (gegebenenfalls substituiertes), Hetaryl (gegebenenfalls substituiertes), Alkyl (gegebenenfalls substituiertes), Cyano, $-CO-R^6$, wobei $R^6$ und $R^2$ durch Brückenglieder verbunden sein können,

$R^2$ für Wasserstoff, Deuterium, Alkyl (gegebenenfalls substituiertes), Aryl (gegebenenfalls substituiertes), $-CH_2-R^6$, $-CO-R^6$, wobei $R^6$ und $R^1$ durch Brückenglieder verbunden sein können,

$R^6$ und $R^2$ bzw. $R^6$ und $R^1$ für Alkylen mit 2 bis 4 C-Atomen steht, das ein- oder mehrfach

substituiert sein kann durch Alkyl, Alkoxy, Aryl, Fluor, Alkoxycarbonyl, wobei eine Methylengruppe auch durch O, S, SO, $SO_2$, NH oder N-Alkyl ersetzt sein kann,

$R^3$ für Wasserstoff, Alkyl (gegebenenfalls substituiertes), Acyl,

$R^4$, $R^5$ für Wasserstoff, Alkyl (gegebenenfalls substituiertes, z.B. Trifluormethyl), Halogen, Alkoxy, Nitro, Acyloxy, Cyano, N-Alkylpiperazino, Alkylsulfonyl, Arylsulfonyl, Carboxyl und wobei $R^4$ und $R^5$ gleich oder verschieden sein können,

$R^3$ für Wasserstoff, Alkyl (gegebenenfalls substituiertes),

$R^4$, $R^5$, für Wasserstoff, Alkyl (gegebenenfalls substituiertes), Halogen, Alkoxy, Nitro, Acyloxy, Cyano, Alkylpiperazino, Alkylsulfonyl, Arylsulfonyl, Amidosulfonyl, Carboxyl, und wobei $R^4$ und $R^5$ gleich oder verschieden sein können, und

$R^6$ für Wasserstoff, Alkyl (gegebenenfalls substituiertes), Aryl (gegebenenfalls substituiertes), Cycloalkyl, Amino (gegebenenfalls substituiertes), gegebenenfalls substituiertes Alkoxy, Cycloalkoxy, Alkoxycarbonyl, gegebenenfalls substituiertes Thioalkyl und Thiophenyl steht,

X für CH oder N steht,

mit einem pharmazeutisch unbedenklichen Verdünnungsmittel oder Trägermaterial vermischt und in eine geeignete Applikationsform überführt.

10. Verbindungen aus der Gruppe bestehend aus:

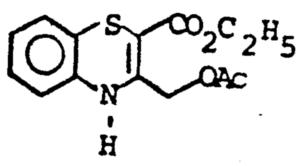

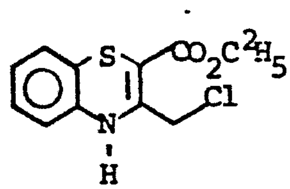

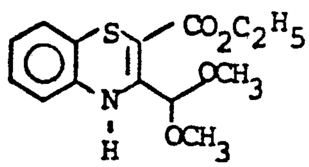

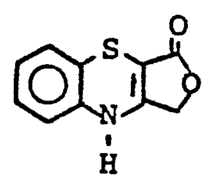

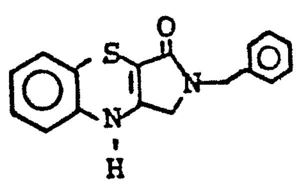

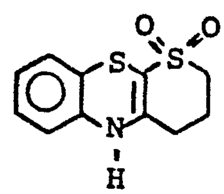

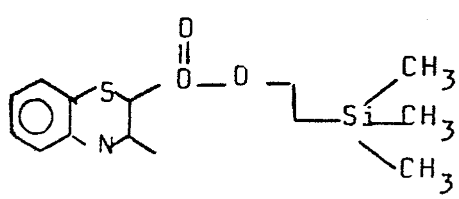

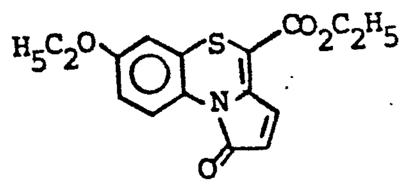

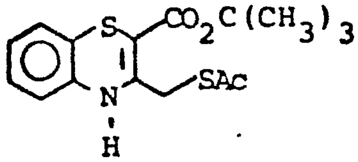

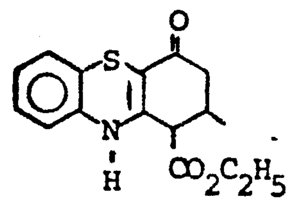

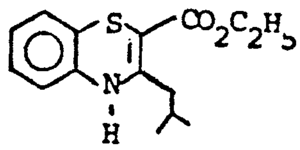

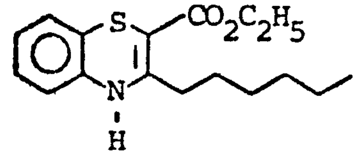

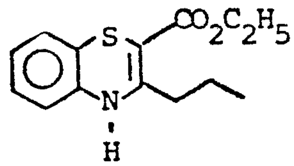

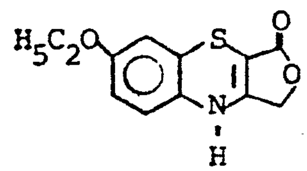

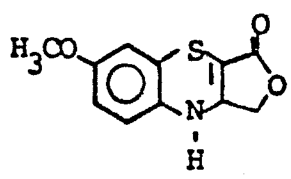

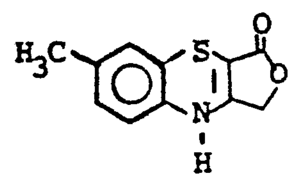

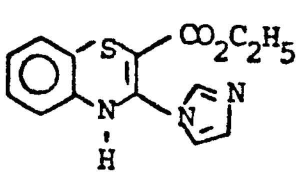

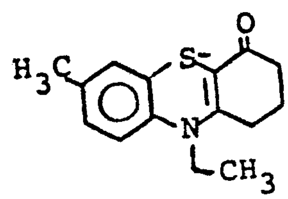

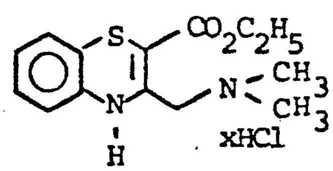

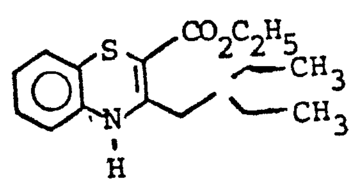

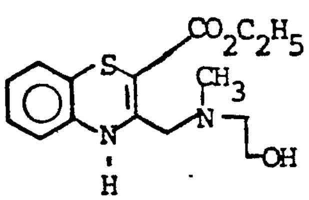

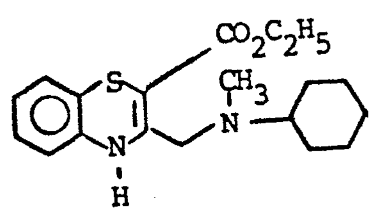

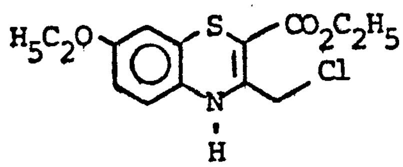

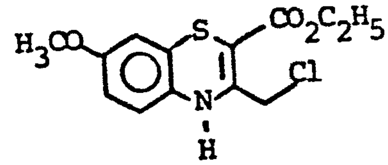

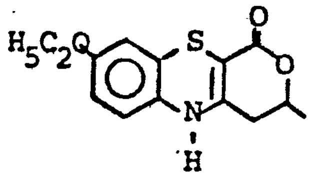

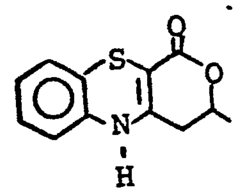

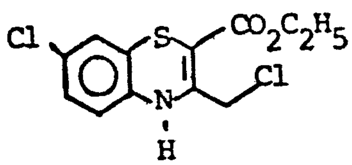

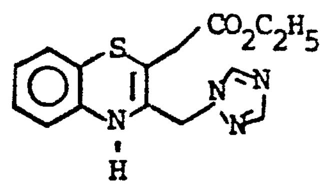

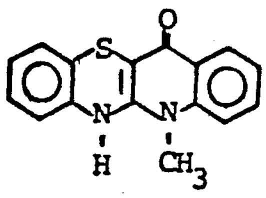

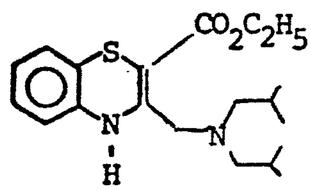

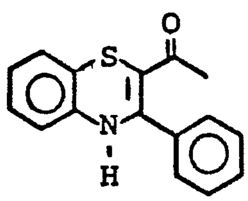

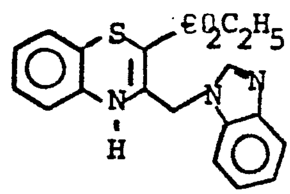

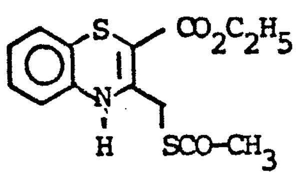

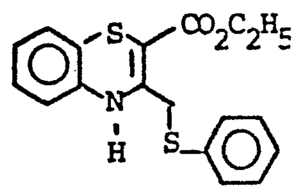

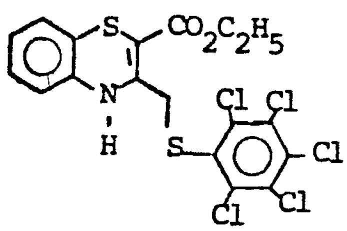

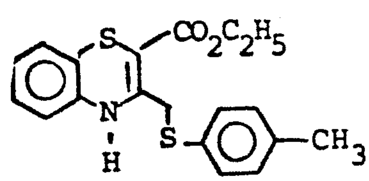

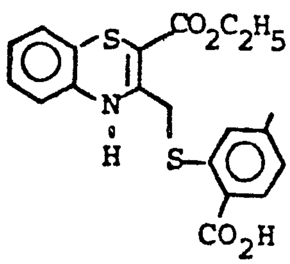

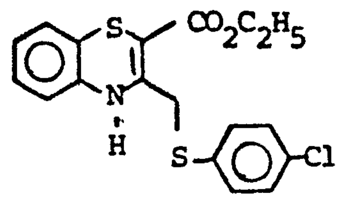

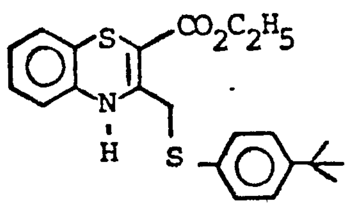

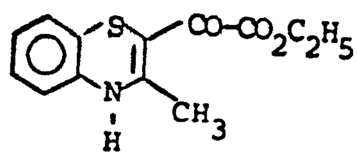

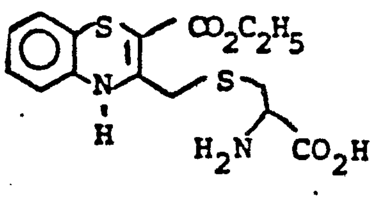

x $H_2O$

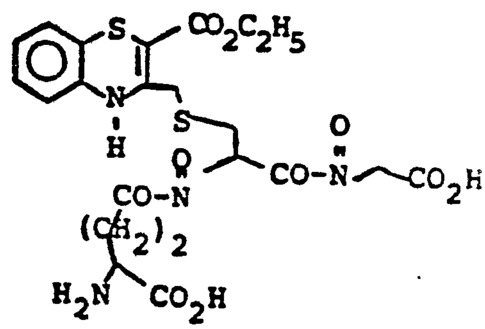

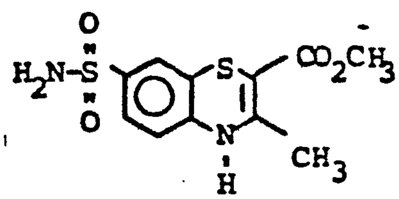

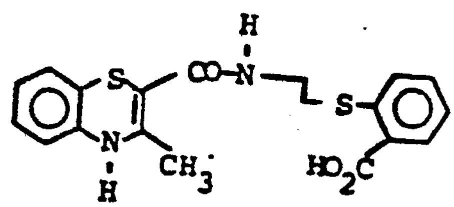

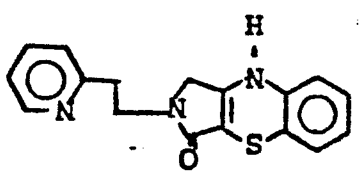

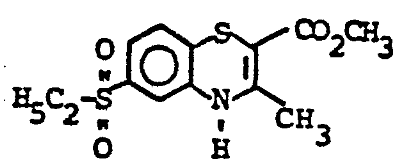

0174458



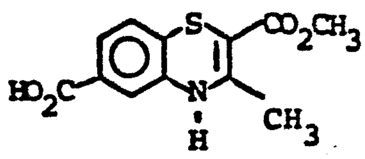

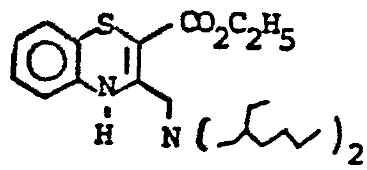

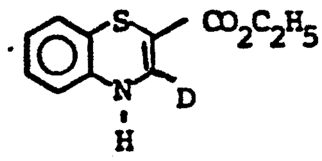

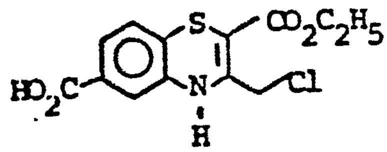

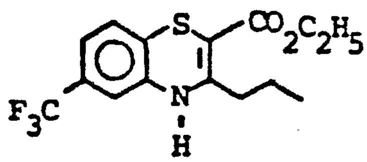

Europäisches Patentamt

# EUROPÄISCHER RECHERCHENBERICHT

0174458
Nummer der Anmeldung

EP 85 10 8578

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl 4) |
|---|---|---|---|
| X | EP-A-0 100 527 (BAYER)<br>* Ansprüche *<br>--- | 1-12 | A 61 K 31/54<br>C 07 D 279/16<br>C 07 D 279/20<br>C 07 D 279/22<br>C 07 D 279/14 |
| X | EP-A-0 101 898 (BAYER)<br>* Ansprüche *<br>--- | 1-12 | C 07 D 513/04<br>C 07 D 417/04<br>C 07 D 417/06<br>C 07 F 7/08 // |
| X | EP-A-0 101 933 (BAYER)<br>* Ansprüche *<br>--- | 1-12 | (C 07 D 513/04<br>C 07 D 311:00<br>C 07 D 279:00 )<br>(C 07 D 513/04 |
| X | CHEMICAL ABSTRACTS, vol. 72, no. 13, 30. März 1970, Seite 406, 66965m, Columbus, Ohio, US; & JP - A - 69 27 588 (FUJISAWA PHARMACEUTICAL CO., LTD.) 15.11.1969<br>* Zusammenfassung *<br>--- | 1 | C 07 D 307:00<br>C 07 D 279:00 )<br>(C 07 D 513/04<br>C 07 D 279:00<br>C 07 D 209:00 )<br>-/- |
| | | | RECHERCHIERTE SACHGEBIETE (Int. Cl 4) |
| Y | CHEMICAL ABSTRACTS, Band 78, Nr. 7, 19. Februar 1973, Seite 485, Nr. 43395k, Columbus, Ohio, US; V.I. SHVEDOV et al.: "Synthesis and reactions of 1,2,3,4-tetrahydrophenothiazines" & KHIM. GETEROTSIKL. SOEDIN. 1972, (11), 1509-13<br>* Zusammenfassung *<br>--- -/- | 12 | A 61 K 31/00<br>C 07 D 279/00<br>C 07 D 513/00<br>C 07 D 417/00<br>C 07 F 7/00 |

Der vorliegende Recherchenbericht wurde für alle Patentansprüche erstellt

| Recherchenort | Abschlußdatum der Recherche | Prüfer |
|---|---|---|
| DEN HAAG | 02-09-1985 | CHOULY J. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1503 03 82

Europäisches Patentamt

**0174458**

# EUROPÄISCHER RECHERCHENBERICHT

Nummer der Anmeldung

EP 85 10 8578

Seite 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| Y | JOURNAL OF THE CHEMICAL SOCIETY, ISSN:0300-922X, JCPRB (3)645-880(1982), 1982, Seiten 831-834, PERKIN TRANSACTIONS I, ORGANIC AND BIO-ORGANIC CHEMISTRY; THOMAS L. GILCHRIST et al.: "1H-1,4-Benzothiazines. New Cyclic Sulphonium Ylides" * Seite 832, Formel 11 * | 12 | (C 07 D 513/04<br>C 07 D 335:00<br>C 07 D 279:00 )<br>(C 07 D 513/04<br>C 07 D 279:00<br>C 07 D 221:00 ) |
| | ----- | | RECHERCHIERTE SACHGEBIETE (Int. Cl.4) |

Der vorliegende Recherchenbericht wurde für alle Patentansprüche erstellt

| Recherchenort | Abschlußdatum der Recherche | Prüfer |
|---|---|---|
| DEN HAAG | 02-09-1985 | CHOULY J. |

KATEGORIE DER GENANNTEN DOKUMENTEN
- X : von besonderer Bedeutung allein betrachtet
- Y : von besonderer Bedeutung in Verbindung mit einer anderen Veröffentlichung derselben Kategorie
- A : technologischer Hintergrund
- O : nichtschriftliche Offenbarung
- P : Zwischenliteratur
- T : der Erfindung zugrunde liegende Theorien oder Grundsätze
- E : älteres Patentdokument, das jedoch erst am oder nach dem Anmeldedatum veröffentlicht worden ist
- D : in der Anmeldung angeführtes Dokument
- L : aus andern Gründen angeführtes Dokument
- & : Mitglied der gleichen Patentfamilie, übereinstimmendes Dokument

EPA Form 1503 03 82